# EUROPEAN PATENT SPECIFICATION

(11) **EP 2 401 400 B1**
(45) Date of publication and mention of the grant of the patent: **06.01.2016**
(21) Application number: 10716040.0
(22) Date of filing: 26.02.2010
(51) Int. Cl.: C12Q 1/68

(54) **METHODS AND COMPOSITIONS FOR EVALUATING CHRONIC IMMUNE DISEASES**
VERFAHREN UND ZUSAMMENSETZUNGEN ZUR BEWERTUNG CHRONISCHER IMMUNERKRANKUNGEN
PROCÉDÉS ET COMPOSITIONS D'ÉVALUATION DE MALADIES IMMUNITAIRES CHRONIQUES

(30) Priority: 27.02.2009 US 156346 P
(43) Date of publication of application: 04.01.2012
(73) Proprietor: Protea Biopharma N.V., 1120 Neder-Over-Heembeek (BE)
(72) Inventor: FREMONT, Marc, B-1731 Zellik (BE); DE MEIRLEIR, Kenny, Leo, B-2800 Mechelen (BE)
(74) Representative: Heaton, Joanne Marie
(86) International application number: PCT/IB2010/000656
(87) International publication number: WO 2010/097706

(56) References cited:
- WO-A1-98/37239
- WO-A1-2009/137686
- WO-A2-2008/010082
- DHIMAN ET AL: "Associations between SNPs in toll-like receptors and related intracellular signaling molecules and immune responses to measles vaccine: Preliminary results" VACCINE, ELSEVIER LTD, GB LNKD- DOI:10.1016/J.VACCINE.2008.01.017, vol. 26, no. 14, 4 February 2008 (2008-02-04), pages 1731-1736, XP022521906 ISSN: 0264-410X
- GUIMARÃES P E M ET AL: "DNA polymorphisms as tools for spinal cord injury research." SPINAL CORD : THE OFFICIAL JOURNAL OF THE INTERNATIONAL MEDICAL SOCIETY OF PARAPLEGIA FEB 2009 LNKD- PUBMED:18504448, vol. 47, no. 2, 27 May 2008 (2008-05-27), pages 171-175, XP008123346 ISSN: 1362-4393 & GUIMARÃES ET AL: "DNA polymorphisms as tools for spinal cord injury research - supplementary material pages 1, 62-65" SPINAL CORD, vol. 47, no. 2, 27 May 2008 (2008-05-27), pages 171-175, XP002587129
- METZGER K ET AL: "Lower frequency of IL-17F sequence variant (His161Arg) in chronic fatigue syndrome patients" BIOCHEMICAL AND BIOPHYSICAL RESEARCH COMMUNICATIONS, ACADEMIC PRESS INC. ORLANDO, FL, US LNKD- DOI:10.1016/J.BBRC.2008.08.135, vol. 376, no. 1, 7 November 2008 (2008-11-07), pages 231-233, XP025465419 ISSN: 0006-291X [retrieved on 2008-09-05]
- GOERTZEL BENJAMIN N ET AL: "Combinations of single nucleotide polymorphisms in neuroendocrine effector and receptor genes predict chronic fatigue syndrome." PHARMACOGENOMICS APR 2006 LNKD- PUBMED:16610957, vol. 7, no. 3, April 2006 (2006-04), pages 475-483, XP002587130 ISSN: 1462-2416
- TORPY D J ET AL: "ASSOCIATION BETWEEN CHRONIC FATIGUE SYNDROME AND THE CORTICOSTEROID-BINDING GLOBULIN GENE ALA SER224 POLYMORPHISM" ENDROCRINE RESEARCH, MARCEL DEKKER, NEW YORK, NY, US LNKD- DOI:10.1081/ERC-200035599, vol. 30, no. 3, 1 August 2004 (2004-08-01), pages 417-429, XP009049832 ISSN: 0743-5800
- LIGHT A R ET AL: "Moderate Exercise Increases Expression for Sensory, Adrenergic, and Immune Genes in Chronic Fatigue Syndrome Patients But Not in Normal Subjects" JOURNAL OF PAIN, SAUNDERS, PHILADELPHIA, PA, US LNKD- DOI:10.1016/J.JPAIN.2009.06.003, vol. 10, no. 10, 1 October 2009 (2009-10-01), pages 1099-1112, XP026661383 ISSN: 1526-5900 [retrieved on 2009-07-31]
- LORENZ E ET AL: "Determination of the TLR4 genotype using allele-specific PCR.", BIOTECHNIQUES JUL 2001, vol. 31, no. 1, July 2001 (2001-07), pages 22-24, ISSN: 0736-6205
- SCHMITT CHRISTOPHER ET AL: "Polymorphisms of TLR4: rapid genotyping and reduced response to lipopolysaccharide of TLR4 mutant alleles.", CLINICAL CHEMISTRY OCT 2002, vol. 48, no. 10, October 2002 (2002-10), pages 1661-1667, ISSN: 0009-9147
- HENCKAERTS L ET AL: "M1149 Genetic Markers and the Risk of Complicated Disease Behaviour in Crohn's Disease Patients", GASTROENTEROLOGY, ELSEVIER, PHILADELPHIA, PA, vol. 134, no. 4, 1 April 2008 (2008-04-01) , pages A-349, XP023433422, ISSN: 0016-5085, DOI: 10.1016/S0016-5085(08)61625-X [retrieved on 2008-04-01]
- IMAHARA ET AL: "The influence of gender on human innate immunity", SURGERY, C.V. MOSBY CO., ST. LOUIS, US, vol. 138, no. 2, 1 August 2005 (2005-08-01), pages 275-282, XP005688186, ISSN: 0039-6060

## Description

### INTRODUCTION

Chronic Fatigue Syndrome (CFS)/myalgic encephalomyelitis is defined by a severe and debilitating fatigue associated with a variety of symptoms including musculoskeletal pain, sore throat, tender lymph nodes, sleep abnormalities, and neurocognitive problems (Fukuda et al. (1994) Ann Intern Med 121:953-959). The pathogenesis of CFS is still poorly understood, but is likely to be multifactorial; viral infections, stress, neuroendocrine dysfunctions, exposure to toxins have all been proposed as contributing factors to the onset and maintenance of the disease (Devanur and Kerr (2006) J Clin Virol 37(3):139-50).

Metzger et al. (2008), Biochemical and biophysical research communications, 376(1): 231-233, discloses a SNP in IL-17F associated with CFS. Dhiman et al. (2008), Vaccine, 26(14), 1731-1736, discloses SNPs in toll-like receptors which are associated with the response to a measles vaccine.

### SUMMARY

Methods and compositions are provided for evaluating a subject for a chronic immune disease. Aspects of the methods include genotyping a subject to determine whether the subject has a least one polymorphism in at least one gene chosen from TLR4, Hsp60, IL-23R, IL-6 and IL-17F. In addition, reagents, devices and kits thereof that find use in practicing the subject methods are provided.

### DETAILED DESCRIPTION

Methods and compositions are provided for evaluating a subject for a chronic immune disease, including predicting whether a subject is susceptible to a chronic immune disease. Aspects of the methods include genotyping a subject to determine whether the subject has a least one polymorphism in at least one gene chosen from TLR4, Hsp60, IL-23R, IL-6 and IL-17F. In addition, reagents, devices and kits thereof that find use in practicing the subject methods are provided.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Methods recited herein may be carried out in any order of the recited events which is logically possible, as well as the recited order of events.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

As summarized above, the subject invention is directed to methods of evaluating chronic immune disease including predicting whether a subject is susceptible to a chronic immune disease, as well as reagents and kits thereof for use in practicing the subject methods. In further describing the invention, the subject methods are described first, followed by a review of the reagents, kits and devices for use in practicing the subject methods.

### METHODS OF EVALUATING A SUBJECT FOR CHRONIC IMMUNE DISEASE

Embodiments of the subject invention are directed to methods for predicting whether a subject is susceptible to Chronic Fatigue Syndrome.

Chronic Fatigue Syndrome (CFS), also known as myalgic encephalomyelitis (ME), is a condition in which the subject suffers from fatigue associated with symptoms which may include but are not limited to muscle and joint (musculoskeletal) pain, sore throat, tender lymph nodes, sleep abnormalities, and/or neurocognitive problems. Often, the symptoms last for six months or more. Often, the fatigue is debilitating. In certain embodiments, the methods are directed at CFS and particularly at whether a subject will or will not be susceptible to CFS, at diagnosing a subject as having CFS, or at determining what treatment to provide a subject with CFS.

Aspects of the invention include methods of predicting whether a subject is or is not susceptible to CFS, or is or is not resistant to CFS. A subject is susceptible to CFS if the subject is more likely to develop CFS than an individual of the general population, whereas a subject that is resistant to CFS is less likely to develop CFS than an individual of the general population.

Methods of diagnosing whether a subject suspected of having CFS has CFS are also described. Subjects suspected of having CFS and thus amenable to these methods can be identified using any convenient protocol. One convenient protocol is diagnosis based on clinical symptoms. A number of different clinical symptoms may be used to identify subjects that may have or have CFS. For example, clinical symptoms of interest include: fatigue of six months or longer that causes a reduction in effort of greater than 50 percent of normal output, pain in multiple joints (arthralgia), muscle pain (myalgia), frequent or recurring sore throat, or tender cervical or axillary lymph nodes, headaches of a new kind or greater severity, cognitive dysfunction (e.g. memory loss, impaired concentration); and the like. The presence of one or more of the above symptoms may be used to identify subjects suspected of having CFS.

The description further describes methods of determining a treatment protocol/regimen for a subject suffering from CFS. These methods may be used to predict the risk of a CFS complication, where that result may be used to determine a treatment protocol. In other words, the methods may be used to predict the risk of a CFS subject developing a complication, the results of such a prediction being useful in determining a prophylactic treatment to prevent the complication. The methods may also be used to diagnose the cause of an existing complication, wherein that result may be used to determine a treatment for that complication. A complication is an unfavorable evolution of a disease, a health condition or a medical treatment. Complications could be a natural evolution of CFS, or a result of a medical treatment that produces adverse effects and/or produces new health problem(s) by itself, or a new disease that develops as a result of CFS. Indications of complications include but are not limited to indications that the CFS has become worse in its severity or shows a increased number of signs, symptoms or new pathologies.

In practicing methods according to certain embodiments, a subject or patient sample, e.g., cells or collections thereof, e.g., tissues, is assayed to determine whether the host or subject from which the assayed sample was obtained is susceptible to CFS, or to diagnose a subject as having CFS, or determine a treatment for a person suffering from CFS. In practicing the subject diagnostic methods, the sample obtained from the host is assayed to determine the genotype of the host or subject from which the sample was obtained with respect to at least one, i.e., one or more, polymorphisms, where polymorphisms of interest are referred to herein as target polymorphisms. In certain embodiments, the at least one target polymorphism is in the TLR4, Hsp60, IL-23R, IL-6 or IL-17F gene.

A TLR4 polymorphism is a polymorphism present in the TLR4 (Toll-like receptor 4) protein. The amino acid sequence for TLR4 and the nucleic acid sequence that encodes it are deposited at Genbank NM_138554 (SEQ ID NO:1 and SEQ ID NO:2). TLR4 is a type I transmembrane protein that acts as a cell surface receptor, for example, as a pattern recognition receptor (PRR) on immune cells for pathogen associated molecular patterns (PAMPs), wherein recognition and binding of PAMPs by TLR4 often induces a pro-inflammatory cellular response. Often, recognition of PAMPs occurs when TLR4 heterodimerizes with another TLR. Polymorphisms of interest include but are not limited to those that modulate TLR4 function such that cellular responsiveness to, e.g. LPS, is diminished, including those that modulate TLR4 recognition of PAMPs, those that modulate dimerization of TLR4 with other TLRs, and those that modulate downstream signaling mechanism by which TLR4 stimulates a cell, for example, the rs4986790 polymorphism (Asp299Gly), wherein a Gly at residue 299 is correlated with a diminished cellular response to LPS and is predictive of susceptibility to CFS. Polymorphisms of particular interest include the rs4986790 A/G polymorphism, in which the A allele encodes a variant with an Asp at residue 299 and the G allele encodes a variant with a Gly at residue 299 ("Asp299Gly"), wherein the G allele is predictive of susceptibility to CFS; and the rs12344353 T/C polymorphism, in which there is a T/C genetic polymorphism in the first intron (i.e. the intron between exons 1 and 2) of the TL4R gene, where the C allele is predictive of susceptibility to CFS.

An Hsp60 polymorphism is a polymorphism present in the Hsp60 (Heat shock protein 60) protein. The amino acid sequence for Hsp60, also known as HSPD1 and chaperonin 60-kD (CPN60), and variants, and the nucleic acid sequences that encode them are deposited at Genbank NM_002156 (SEQ ID NO:3 and SEQ ID NO:4) and NM_199440 (SEQ ID NO:5 and SEQ ID NO:6). Hsp60 is a molecular chaperone, acting, for example, to assist in protein folding and in replication and transmission of mitochondrial DNA. Polymorphisms of interest include but are not limited to those that modulate Hsp60 function, for example, by modulating binding with proteins they are chaperoning, by modulating binding with ATP, by modulating binding with chaperonin proteins, or by modulating binding with single strand DNA. Polymorphisms of particular interest include the rs2340690 T/C polymorphism, in which there is a T/C genetic polymorphism in an intron of the Hsp60 gene, where two T alleles is predictive of susceptibility to CFS.

An IL-23R polymorphism is a polymorphism present in the IL-23R (interleukin 23 receptor) protein. The amino acid sequence for IL-23R and the nucleic acid sequence that encodes it are deposited at Genbank NM_144701 (SEQ ID NO:7 and SEQ ID NO:8). IL-23R is a type 1 transmembrane protein that acts as a receptor for cytokines including IL-23, wherein receptor ligand binding by IL-23R often induces a pro-inflammatory response. Polymorphisms of interest include but are not limited to those that modulate IL-23R function, for example, by modulating IL-23R binding to its ligands or by modulating downstream signaling. Polymorphisms of particular interest include the rs11209026 G/A polymorphism, wherein the G allele encodes a variant with an Arg at residue 381 and the A allele encodes a variant with a Gin at residue 381 ("Arg381Gln", or "R381Q"), where the A allele is predictive of susceptibility to CFS.

An IL-6 polymorphism is a polymorphism present in the IL-6 (interleukin 6) protein. The amino acid amino acid sequence for IL-6, also known as interferon beta-2 (IFNβ2), B-cell stimulatory factor (BSF2), hepatocyte stimulatory factor (HSF), and hybridoma growth factor (HGF), and the nucleic acid sequence that encodes it are deposited at Genbank NM_000600 (SEQ ID NO:9 and SEQ ID NO:10). IL-6 is a cytokine that often produces a proinflammatory cellular response. Polymorphisms of interest include but are not limited to those which modulate IL-6 function, for example, by increasing IL-6 binding to its receptor or increasing IL-6 levels in the blood, as with, for example, the rs1800795 polymorphism, in which the G allele enhances promoter activity, resulting in increased levels of IL-6 in serum. Polymorphisms of particular interest include the rs1800795 C/G polymorphism, in which there is a C/G genetic polymorphism in the promoter region of the IL-6 gene, where the G allele is predictive of susceptibility to CFS.

An IL-17F polymorphism is a polymorphism present in the IL-17F (Interleukin 17F) protein. The amino acid sequence for IL-17F the nucleic acid sequence that encodes it are deposited at Genbank NM_052872 (SEQ ID NO:11 and SEQ ID NO:12). IL-17F is a cytokine that often produces a proinflammatory response. Polymorphisms of interest include but are not limited to polymorphisms that encode amino acid substitutions that modulate IL-17F function, for example, by antagonizing IL-17F function, e.g. proinflammatory effects, e.g. by diminishing IL-17F binding to its receptor or reducing IL-17F levels in the blood such that cellular response is diminished, as with, for example, the rs763780 polymorphism, in which an Arg at residue 161 is associated with diminished cellular response. Polymorphisms of particular interest include the rs763780 T/C polymorphism, wherein the T allele encodes a variant with a His at residue 161 and the C allele encodes a variant with an Arg at residue 161 ("His161Arg" or "H161 R"), where the C allele confers resistance to CFS.

More information about the aforementioned polymorphisms as well as other polymorphisms in the aforementioned genes that may be of interest in the present invention may be found in the NCBI SNP database, searchable at the website having an address produced by placing "http://www." in front of "ncbi.nlm.nih.gov/PubMed/", using the polymorphism or the gene name as the search term and clicking on the link to the "gene view" in any entry.

In certain embodiments, the sample is assayed to determine the genotype of the host with respect to a single target polymorphism. In these embodiments, the single target polymorphism is a polymorphism in the TLR4, Hsp60, IL-23R, IL-6 or IL-17F gene. In certain embodiments, the sample is assayed to determine the genotype of the host with respect to two or more different target polymorphisms, where in these embodiments, the two or more different target polymorphisms are polymorphisms in the TLR4, Hsp60, IL-23R, IL-6 or IL-17F genes. In certain of these embodiments, at least two of the target polymorphisms are polymorphisms in different genes. In certain embodiments, the sample is assayed for both a first and a second polymorphism

Any convenient protocol for assaying a sample for the above one or more target polymorphisms may be employed in the subject methods. In certain embodiments, the target polymorphism is detected at the protein level, e.g., by assaying for a polymorphic protein. In yet other embodiments, the target polymorphism is detected at the nucleic acid level, e.g., by assaying for the presence of nucleic acid polymorphism, e.g., a single nucleotide polymorphism (SNP) that causes expression of the polymorphic protein.

For example, polynucleotide samples derived from (e.g., obtained from) an individual may be employed. Any biological sample that comprises a polynucleotide from the individual is suitable for use in the methods of the invention. The biological sample may be processed so as to isolate the polynucleotide. Alternatively, whole cells or other biological samples may be used without isolation of the polynucleotides contained therein. Detection of a target polymorphism in a polynucleotide sample derived from an individual can be accomplished by any means known in the art, including, but not limited to, amplification of a sequence with specific primers; determination of the nucleotide sequence of the polynucleotide sample; hybridization analysis; single strand conformational polymorphism analysis; denaturing gradient gel electrophoresis; mismatch cleavage detection; and the like. Detection of a target polymorphism can also be accomplished by detecting an alteration in the level of a mRNA transcript of the gene; aberrant modification of the corresponding gene, e.g., an aberrant methylation pattern; the presence of a non-wild-type splicing pattern of the corresponding mRNA; an alteration in the level of the corresponding polypeptide; and/or an alteration in corresponding polypeptide activity.

Detection of a target polymorphism by analyzing a polynucleotide sample can be conducted in a number of ways. A test nucleic acid sample can be amplified with primers which amplify a region known to comprise the target polymorphism(s). Genomic DNA or mRNA can be used directly. Alternatively, the region of interest can be cloned into a suitable vector and grown in sufficient quantity for analysis. The nucleic acid may be amplified by conventional techniques, such as a polymerase chain reaction (PCR), to provide sufficient amounts for analysis. The use of the polymerase chain reaction is described in a variety of publications, including, e.g., "PCR Protocols (Methods in Molecular Biology)" (2000) J.M.S. Bartlett and D. Stirling, eds, Humana Press; and "PCR Applications: Protocols for Functional Genomics" (1999) Innis, Gelfand, and Sninsky, eds., Academic Press. Once the region comprising a target polymorphism has been amplified, the target polymorphism can be detected in the PCR product by nucleotide sequencing, by Single Strand Conformation Polymorphism (SSCP) analysis, or any other method known in the art. In performing SSCP analysis, the PCR product may be digested with a restriction endonuclease that recognizes a sequence within the PCR product generated by using as a template a reference sequence, but does not recognize a corresponding PCR product generated by using as a template a variant sequence by virtue of the fact that the variant sequence no longer contains a recognition site for the restriction endonuclease. PCR may also be used to determine whether a polymorphism is present by using a primer that is specific for the polymorphism. Such methods may comprise the steps of collecting from an individual a biological sample comprising the individual's genetic material as template, optionally isolating template nucleic acid (genomic DNA, mRNA, or both) from the biological sample, contacting the template nucleic acid sample with one or more primers that specifically hybridize with a target polymorphic nucleic acid molecule under conditions such that hybridization and amplification of the template nucleic acid molecules in the sample occurs, and detecting the presence, absence, and/or relative amount of an amplification product and comparing the length to a control sample. Observation of an amplification product of the expected size is an indication that the target polymorphism contained within the target polymorphic primer is present in the test nucleic acid sample. Parameters such as hybridization conditions, polymorphic primer length, and position of the polymorphism within the polymorphic primer may be chosen such that hybridization will not occur unless a polymorphism present in the primer(s) is also present in the sample nucleic acid. Those of ordinary skill in the art are well aware of how to select and vary such parameters. See, e.g., Saiki et al. (1986) Nature 324:163; and Saiki et al (1989) Proc. Natl. Acad. Sci. USA 86:6230. As one non-limiting example, a PCR primer comprising the T78C variation described in Example 1 may be used.

Alternatively, various methods are known in the art that utilize oligonucleotide ligation as a means of detecting polymorphisms. See, e.g., Riley et al. (1990) Nucleic Acids Res. 18:2887-2890; and Delahunty et al. (1996) Am. J. Hum. Genet. 58:1239-1246.

A detectable label may be included in an amplification reaction. Suitable labels include fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may be a two stage system, where the amplified DNA is conjugated to biotin, haptens, etc. having a high affinity binding partner, e.g. avidin, specific antibodies, etc., where the binding partner is conjugated to a detectable label. The label may be conjugated to one or both of the primers. Alternatively, the pool of nucleotides used in the amplification is labeled, so as to incorporate the label into the amplification product.

The sample nucleic acid may be sequenced by a dideoxy chain termination method or other well-known methods. Genomic DNA or mRNA may be used directly. If mRNA is used, a cDNA copy may first be made. If desired, the sample nucleic acid can be amplified using a PCR. A variety of sequencing reactions known in the art can be used to directly sequence the relevant gene, or a portion thereof in which a specific polymorphism is known to occur, and detect polymorphisms by comparing the sequence of the sample nucleic acid with a reference polynucleotide that contains a target polymorphism. Any of a variety of automated sequencing procedures can be used. See, e.g., WO 94/16101; Cohen et al. (1996) Adv. Chromatography 36:127-162.

Hybridization with the variant sequence may also be used to determine the presence of a target polymorphism. Hybridization analysis can be carried out in a number of different ways. including, but not limited to Southern blots, Northern blots, dot blots, microarrays, etc. The hybridization pattern of a control and variant sequence to an array of oligonucleotide probes immobilized on a solid support, as described in U.S. 5,445,934, or in WO 95/35505, may also be used as a means of detecting the presence of variant sequences. Identification of a polymorphism in a nucleic acid sample can be performed by hybridizing a sample and control nucleic acids to high density arrays containing hundreds or thousands of oligonucleotide probes. Cronin et al. (1996) Human Mutation 7:244-255; and Kozal et al. (1996) Nature Med. 2:753-759.

Single strand conformational polymorphism (SSCP) analysis; denaturing gradient gel electrophoresis (DGGE); mismatch cleavage detection; and heteroduplex analysis in gel matrices can also be used to detect polymorphisms. Alternatively, where a polymorphism creates or destroys a recognition site for a restriction endonuclease (restriction fragment length polymorphism, RFLP), the sample is digested with that endonuclease, and the products size fractionated to determine whether the fragment was digested. Fractionation is performed by gel or capillary electrophoresis, particularly acrylamide or agarose gels. The aforementioned techniques are well known in the art. Detailed description of these techniques can be found in a variety of publications, including, e.g., "Laboratory Methods for the Detection of Mutations and Polymorphisms in DNA" (1997) G.R. Taylor, ed., CRC Press, and references cited therein.

A number of methods are available for determining the expression level of a polymorphic nucleic acid molecule, e.g., a polymorphic polypeptide in a particular sample. Diagnosis may be performed by a number of methods to determine the absence or presence or altered amounts of normal or abnormal mRNA in a patient sample. For example, detection may utilize staining of cells or histological sections with labeled antibodies, performed in accordance with conventional methods. Cells are permeabilized to stain cytoplasmic molecules. The antibodies of interest are added to the cell sample, and incubated for a period of time sufficient to allow binding to the epitope, usually at least about 10 minutes. The antibody may be labeled with radioisotopes, enzymes, fluorescers, chemiluminescers, or other labels for direct detection. Alternatively, a second stage antibody or reagent is used to amplify the signal. Such reagents are well known in the art. For example, the primary antibody may be conjugated to biotin, with horseradish peroxidase-conjugated avidin added as a second stage reagent. Alternatively, the secondary antibody conjugated to a fluorescent compound, e.g. fluorescein, rhodamine, Texas red, *etc.* Final detection uses a substrate that undergoes a color change in the presence of the peroxidase. The absence or presence of antibody binding may be determined by various methods, including flow cytometry of dissociated cells, microscopy, radiography, scintillation counting, *etc.* Detailed descriptions of how to make antibodies can be found in a variety of publications, including, e.g. "Making and using Antibodies: A Practical Handbook" (2006) G.C. Howard and M.R. Kaser, eds. CRC Press; "Antibody Engineering: Methods and Protocols" (2004) B.K.C. Lo, ed, Humana Press.

Alternatively, one may focus on the expression of mRNA. Biochemical studies may be performed to determine whether a sequence polymorphism in a coding region or control regions of interest is associated with disease. Disease associated polymorphisms may include deletion or truncation of the gene, mutations that alter expression level, that affect the activity of the protein, *etc.*

Alternatively, one may screen for polymorphisms at the protein level. Screening for mutations in a polymorphic polypeptide may be based on the functional or antigenic characteristics of the protein. Functional assays include cofactor binding assays, enzyme activity assays, substrate binding assays or surface expression assays. For example, protein truncation assays are useful in detecting deletions that may affect the biological activity of the protein. The activity of the encoded a polymorphic polypeptide may be determined by comparison with a reference polypeptide lacking a specific polymorphism. Alternatively, the three-dimensional structure of the protein may be assayed, for example by fluorescence polarization or circular dichroism spectroscopy, wherein the three-dimensional structure of the encoded a polymorphic polypeptide may be determined by comparison with purified protein carrying the opposing allele of the polymorphism. Alternatively, various immunoassays designed to detect polymorphisms in polymorphic polypeptides may be used. The absence or presence of antibody binding to a polymorphic polypeptide may be determined by various methods, including flow cytometry of dissociated cells, microscopy, radiography, scintillation counting, *etc.* Detailed descriptions of how to make antibodies, including antibodies that are specific for epitopes, for example, single amino acid substitutions within epitopes, can be found in a variety of publications, including, e.g. "Making and using Antibodies: A Practical Handbook" (2006) G.C. Howard and M.R. Kaser, eds. CRC Press; "Antibody Engineering: Methods and Protocols" (2004) B.K.C. Lo, ed, Humana Press; and US Patent No. 6,054,632.

Diagnostic methods of the subject invention in which the level of polymorphic gene expression is of interest will typically involve comparison of the relevant nucleic acid abundance of a sample of interest with that of a control value to determine any relative differences, where the difference may be measured qualitatively and/or quantitatively, which differences are then related to the presence or absence of an abnormal gene expression pattern. A variety of different methods for determining the nucleic acid abundance in a sample are known to those of skill in the art, where particular methods of interest include those described in: Pietu et al., Genome Res. (June 1996) 6: 492-503; Zhao et al., Gene (April 24, 1995) 156: 207-213; Soares, Curr. Opin. Biotechnol. (October 1997) 8: 542-546; Raval, J. Pharmacol Toxicol Methods (November 1994) 32: 125-127; Chalifour et al., Anal. Biochem (February 1, 1994) 216: 299-304; Stolz & Tuan, Mol. Biotechnol. (December 19960 6: 225-230; Hong et al., Bioscience Reports (1982) 2: 907; and McGraw, Anal. Biochem. (1984) 143: 298. Also of interest are the methods disclosed in WO 97/2731 7.

Additional references describing various protocols for detecting the presence of a target polymorphism include, but are not limited to, those described in: US Patent Nos. 6,703,228; 6,692,909; 6,670,464; 6,660,476; 6,653,079; 6,632,606; 6,573,049.

Following obtainment of the genotype from the sample being assayed, the genotype is evaluated to determine if the subject is susceptible/resistant to CFS. In certain embodiments, the obtained genotype may be compared with a reference or control to make a diagnosis regarding the CFS phenotype of the cell or tissue, and therefore host, from which the sample was obtained/derived. The terms "reference" and "control" as used herein mean a standardized genotype to be used to interpret the genotype of a given patient and assign a prognostic class thereto. The reference or control may be a genotype that is obtained from a cell/tissue known to have a particular phenotype, e.g., a susceptibility phenotype, and therefore may be a positive reference or control genotype. Alternatively, the reference/control genotype may be from a cell/tissue known to not have the desired phenotype, and therefore be a negative reference/control genotype.

In certain embodiments, the obtained genotype is compared to a single reference/control genotype to obtain information regarding the phenotype of the cell/tissue being assayed. In yet other embodiments, the obtained genotype is compared to two or more different reference/control profiles to obtain more in depth information regarding the phenotype of the assayed cell/tissue. For example, the obtained genotype may be compared to a positive genotype and a negative genotype to obtain confirmed information regarding whether the cell/tissue has the phenotype of interest.

Representative examples of genotypes associated with susceptibility to or with having CFS include, but are not limited to: the G allele of TLR4 polymorphism rs4986790, the C allele of TLR4 polymorphism rs12344353, two T alleles of Hsp60 polymorphism rs2340690, the A allele of IL-23R polymorphism rs11209026, and two G alleles of the IL-6 polymorphism rs1800795. Representative examples of genotypes associated with resistance to CFS include, but are not limited to the C allele of the IL-17F polymorphism rs763780.

The above-obtained information about the cell/tissue being assayed is employed to determine if the subject is susceptible/resistant to CFS. In certain embodiments, the above-obtained information is employed to give a refined probability determination as to whether a subject is susceptible to CFS or to give a refined determination as to whether a subject suffering from CFS will develop a particular complication. For example, an identification of at least one G allele of TLR4 polymorphism rs4986790, at least one C allele of TLR4 polymorphism rs12344353, two T alleles of Hsp60 polymorphism rs2340690, at least one A allele of IL-23R polymorphism rs11209026, or two G alleles of the IL-6 polymorphism rs1800795 may be employed to determine that the subject has at least a 70% chance, such as at least a 75% chance, including at least an 80% chance of developing CFS or a complication from CFS. Likewise, an identification of at least one C allele of the IL-17F polymorphism rs763780 may be employed to determine that the subject has less than 50% chance, such as a less than 45% chance, including a less than 40% chance of developing CFS or a complication from CFS.

In some embodiments, determining that the subject is susceptible/resistant to CFS, or predicting if a subject suffering from CFS will develop a particular complication may be made by employing the results of the aforementioned genotype analysis in conjunction with the identification of symptoms known in the art to be associated with CFS, e.g. a new onset (not lifelong) of unexplained, persistent fatigue unrelated to exertion and not substantially relieved by rest, impaired memory or concentration, post-exertional malaise, where physical or mental exertions bring on "extreme, prolonged exhaustion and sickness", unrefreshing sleep, muscle pain (myalgia), pain in multiple joints (arthralgia), headaches of a new kind or greater severity, frequent or recurring sore throat, or tender cervical or axillary lymph nodes. In addition, the artisan may consider the presence of symptoms that are attributable to other conditions, so as to exclude a determination of susceptibility to CFS or a CFS diagnosis, e.g. symptoms of mononucleosis, Lyme disease, lupus, multiple sclerosis, fibromyalgia, primary sleep disorders, severe obesity and major depressive disorders, as they are known in the art.

In some embodiments, the above-obtained information about the cell/tissue being assayed is employed to determine a treatment for a subject suffering from CFS. Treatments of interest include, but are not limited to, antihistamines, prescription sleep medications, analgesics, anti-depressants, dietary supplements, alternative medicine, cognitive behavior therapy (a form of psychological therapy used to treat chronically ill subjects), graded exercise therapy (a form of physical therapy), and pacing (an energy management strategy in which subjects are advised to set manageable daily activity/exercise goals and balance activity and rest to avoid over-exertion which may worsen symptoms). Classes of therapeutic treatments of interest include, but are not limited to, therapeutic antibodies, peptide mimetics, small molecule agonists or antagonists, siRNA-based therapeutics, and gene therapy.

In some instances, the treatment may be a pharmacogenomic treatment. In these applications, a subject/host/patient is first diagnosed for the presence of absence of a CFS phenotype using a protocol such as the diagnostic protocol described in the preceding section. The subject is then treated using a pharmacological protocol, where the suitability of the protocol for a particular subject/patient is determined using the results of the diagnosis step. In other words, the therapeutic protocol employed is a protocol that is tailored to the particular polymorphism identified in the patient sample. More specifically, where the identified phenotype is a polymorphism in one gene, a particular therapeutic treatment protocol is then employed to treat the subject. Alternatively, where a subject is identified as having a polymorphism in another gene, a different therapeutic treatment protocol is then employed. For example, a therapy for CFS or a CFS complication in a subject with a TLR4 polymorphism may include augmenting TLR4 signaling, for example with peptide mimetic or antibody therapy to promote signaling via the TLR4 Gly variant. As another example, a therapy for CFS or a CFS complication in a subject with an IL-6 rs18000795 polymorphism may include attenuating IL-6 signaling, for example with an IL-6 or IL-6 receptor blocking antibody, an IL-6-specific or IL-6 receptor-specific siRNA, or an IL-6 or IL-6 dominant negative peptide.

In some embodiments, providing an evaluation of a subject for CFS, e.g. a prediction of a subject's susceptibility to developing CFS, includes generating a written report that includes the artisan's assessment of the subject's susceptibility to developing CFS, i.e. a "susceptibility assessment", the subject's current state of health i.e. a "CFS diagnosis assessment", and/or possible treatment regimens, i.e. a "treatment assessment". Thus, a subject method may further include a step of generating or outputting a report providing the results of a susceptibility assessment, a CFS diagnosis assessment, or treatment assessment, which report can be provided in the form of an electronic medium (e.g., an electronic display on a computer monitor), or in the form of a tangible medium (e.g., a report printed on paper or other tangible medium).

A "report," as described herein, is an electronic or tangible document which includes report elements that provide information of interest relating to a susceptibility assessment, a CFS diagnosis assessment, or treatment assessment and its results. A subject report can be completely or partially electronically generated. A subject report includes at least a susceptibility prediction, i.e. a prediction as to the susceptibility of a subject to developing CFS; or a CFS diagnosis, i.e. whether a subject does or does not have CFS; or a suggested treatment regimen, i.e. a course of treatment to be followed. A subject report can further include one or more of: 1) information regarding the testing facility; 2) service provider information; 3) subject data; 4) sample data; 5) an assessment report, which can include various information including: a) test data, where test data can include genotypes for one or more polymorphism in at least one gene chosen from TLR4, Hsp60, IL-23R, IL-6 and IL-17F and b) reference values employed, if any; 6) other features.

The report may include information about the testing facility, which information is relevant to the hospital, clinic, or laboratory in which sample gathering and/or data generation was conducted. Sample gathering can include obtaining a fluid sample, e.g. blood, saliva, urine etc.; or a tissue sample, e.g. a tissue biopsy, a swab of the lining of the mouth or nose, a collect hair follicle, etc. from a subject. Data generation can include genotyping for one or more polymorphisms in TLR4, Hsp60, IL-23R, IL-6 and/or IL-17F. This information can include one or more details relating to, for example, the name and location of the testing facility, the identity of the lab technician who conducted the assay and/or who entered the input data, the date and time the assay was conducted and/or analyzed, the location where the sample and/or result data is stored, the lot number of the reagents (e.g., kit, etc.) used in the assay, and the like. Report fields with this information can generally be populated using information provided by the user.

The report may include information about the service provider, which may be located outside the healthcare facility at which the user is located, or within the healthcare facility. Examples of such information can include the name and location of the service provider, the name of the reviewer, and where necessary or desired the name of the individual who conducted sample gathering and/or data generation. Report fields with this information can generally be populated using data entered by the user, which can be selected from among pre-scripted selections (e.g., using a drop-down menu). Other service provider information in the report can include contact information for technical information about the result and/or about the interpretive report.

The report may include a subject data section, including subject medical history as well as administrative subject data (that is, data that are not essential to the susceptibility prediction, CFS diagnosis, or proposed treatment regimen) such as information to identify the subject (e.g., name, subject date of birth (DOB), gender, mailing and/or residence address, medical record number (MRN), room and/or bed number in a healthcare facility), insurance information, and the like), the name of the subject's physician or other health professional who ordered the susceptibility prediction and, if different from the ordering physician, the name of a staff physician who is responsible for the subject's care (e.g., primary care physician).

The report may include a sample data section, which may provide information about the biological sample analyzed in the susceptibility prediction, such as the source of biological sample obtained from the subject (e.g. blood, saliva, type of tissue, etc.), how the sample was handled (e.g. storage temperature, preparatory protocols) and the date and time collected. Report fields with this information can generally be populated using data entered by the user, some of which may be provided as pre-scripted selections (e.g., using a drop-down menu).

The report may include an assessment report section, which may include information generated after processing of the data as described herein. The interpretive report can include a prediction of the likelihood that the patient will develop CFS. The interpretive report can include, for example, results of genotyping for a TLR4 polymorphism; and/or results of genotyping for an Hsp60 polymorphism; and/or results of genotyping for a IL-23R polymorphism; and/or results of genotyping for a IL-6 polymorphism; and/or results of genotyping for a IL-17F polymorphism; and interpretation, i.e. prediction. The assessment portion of the report can optionally also include a Recommendation(s). For example, where the results indicate an increased susceptibility to developing CFS, the recommendation can include a recommendation that diet or lifestyle be altered or medical intervention be provided as recommended in the art.

It will also be readily appreciated that the reports can include additional elements or modified elements. For example, where electronic, the report can contain hyperlinks which point to internal or external databases which provide more detailed information about selected elements of the report. For example, the patient data element of the report can include a hyperlink to an electronic patient record, or a site for accessing such a patient record, which patient record is maintained in a confidential database. This latter embodiment may be of interest in an in-hospital system or in-clinic setting. When in electronic format, the report is recorded on a suitable physical medium, such as a computer readable medium, e.g., in a computer memory, zip drive, CD, DVD, etc.

It will be readily appreciated that the report can include all or some of the elements above, with the proviso that the report generally includes at least the elements sufficient to provide the analysis requested by the user (e.g., susceptibility prediction).

### REAGENTS, DEVICES AND KITS

Also described are reagents, devices and kits thereof for practicing one or more of the above-described methods. The subject reagents, devices and kits thereof may vary greatly. Reagents and devices of interest include those mentioned above with respect to the methods of identifying the presence of the target polymorphisms, where such reagents may include nucleic acid primers, arrays of nucleic acid probes, antibodies to polymorphic polypeptides (e.g., immobilized on a substrate), signal producing system reagents, etc., depending on the particular detection protocol to be performed. For example, reagents may include PCR primers that are specific for one or more of the genes TLR4, Hsp60, IL-23R, IL-6 and IL-17F or one or more polymorphisms thereof, as described above. Other examples of reagents include arrays that comprise probes that are specific for one or more of the genes of interest or one or more polymorphisms thereof, and antibodies to epitopes of the proteins encoded by these genes of interest wherein the epitope comprises a polymorphism of interest.

In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

The following examples are offered by way of illustration and not by way of limitation.

### EXPERIMENTAL

### EXAMPLE 1

### Subjects and methods

89 CFS patients (40±12 year old) were enrolled in the study. All were diagnosed for CFS according to the clinical criteria of Fukuda et al. ((1994) Ann. Intern. Med. 121:953-959). 56 aged-matched controls (37±12 year old) were also recruited. 79% of the patients, and 70% of the controls, were females. All subjects were of European origin.

Genomic DNA was extracted from whole blood using the Blood and Tissue DNA extraction kit, Qiagen (Venlo, Netherlands). Analysis of the rs763780 polymorphism in the IL-17F gene was performed with the SNPlex™ genotyping technology, Applied Biosystems (Foster City, CA, USA). Genotyping analyses were performed by DNAvision S.A. (Charleroi, Belgium), using ISO17025-accredited procedures.

### Results

A significantly lower prevalence of the His161Arg variant was observed in the CFS population as compared to the control population. 8 out of 89 patients have a CT genotype (8.9%), versus 14 out of 56 controls (25%). None of the CFS patients have the CC genotype, versus 2 out of 56 controls (3.6%). In total, 28.5% of the controls have a CC or CT genotype versus only 8.9% of the patients (OR=4.05, p=0.0018).

The observed lower frequency suggests an involvement of IL-17F, and more generally of Th17 cells, in the pathophysiology of CFS. The His161Arg variant antagonizes the pro-inflammatory effects of wild-type IL-17F, and thereby exerts a protective effect against asthma (Kawaguchi et al. (2006) J. Allergy Clin. Immunol. 117(4):795-801). Accordingly, the development and/or maintenance of CFS involves an increased production of IL-17F, and that expression of inactive variants such as His161Arg confer protection against the disease.

Increased IL-17F production has been observed in various inflammatory situations (Kolls and Linden (2004) Immunity 21 (4):467-476; Kawaguchi et al. (2006) J. Allergy Clin. Immunol. 117(4):795-801). Interestingly, it also occurs in the context of intestinal disease, such as Crohn's disease or ulcerative colitis (Seiderer et al. (2008) Inflamm. Bowel Dis. 14(4):437-445). Intestinal dysfunction is a common symptom in CFS. Alterations of the intestinal microbial flora have been reported (Logan et al. (2003) Med. Hypotheses 60(6) 915-923); such alterations can lead to intestinal mucosal dysfunction, increased intestinal permeability (leaky gut), etc. that will cause an immune response to the LPS of gram-negative enterobacteria (Maes et al. (2007) J. Affect. Disord. 99(1-3):237-240; Maes et al. (2007) Neuro. Endocrinol. Lett. 28(6):739-44). Exposure of immune cells to LPS could therefore be the clue to the chronic immune activation observed in CFS patients. Considering this hypothesis, it is particularly noteworthy that in addition to IL-6 and TGB-β, optimal Th17 cell induction requires the action of Toll-like receptor (TLR)-activated peripheral blood mononuclear cells (PBMCs). TLR-activated monocytes may contribute to Th17 cell induction by cell-cell contact, through ligation of the T-cell receptor (Evans et al. (2007) Proc. Natl. Acad. Sci. USA 104(43):17034-9); alternatively, TLR-activated PBMCs may secrete a specific set of cytokines that will potentiate Th17 induction (Kattah et al. (2008) Arthritis Rheum. 58(6):1619-29). In this last report, it was shown that TLR-4 (receptor for LPS), as well as TLR-7/8, evoked the most robust induction of Th17, whereas stimulation of TLR-1, -2, -3 or -9 was not efficient.

Th17 cell induction could therefore be the link between exposure to enterobacterial LPS and the symptoms of chronic inflammation associated with CFS. Interestingly, Th17 and Th1 responses are mutually exclusive, since IFN-γ suppresses IL-17 and vice-versa (Kolls and Linden (2004) Immunity 21(4):467-476). Th17 induction is therefore consistent with a decreased production of Th1 cytokines, as seen in CFS. The pro-inflammatory effects of Th17-secreted cytokines are also consistent with other specific dysfunctions observed in CFS patients: IL-17 and IL-22 can disrupt the blood-brain barrier; Th17 lymphocytes transmigrate across the blood-brain barrier endothelial cells and promote inflammation of the central nervous system (Kebir et al. (2007) Nat. Med. 13(10):1173-1175). Blood-brain barrier permeability and CNS inflammation is thought to be a key aspect in the pathogenesis of CFS (Bested et al. (2001) Med. Hypotheses 57(2):231-237).

The implication of Th17 cell activation in the pathogenesis of CFS would be a significant progress in the understanding of the disease, opening new therapeutic perspectives. Other IL-17F-related genes, such as IL-23R, may also play a role in the pathogenesis. IL-17F is indeed specifically produced by IL-23R-expressing Th17 cells; IL-23R is also polymorphic and some variants of this receptor predispose to inflammatory bowel disease (Maloy (2008) J. Intern. Med. 263(6):584-590; Seiderer et al. (2008) Inflamm. Bowel Dis. 14(4):437-445). An association between CFS and polymorphisms of the IL-23R gene, or other functionally related genes, would provide additional support for the implication of the IL-17 axis in the pathogenesis of CFS.

### EXAMPLE 2

### Subjects and Methods

### A. Study subjects

Study subjects were 107 patients, 40±12 year old, previously diagnosed for CFS according to the clinical criteria of Fukuda et al. (Fukuda et al. 1994; Ann Intern Med 121:953-959), and 64 healthy controls (non-CFS subjects), 37±12 year old. 79% of patients, and 70% of controls, were females. All subjects were of European origin. Patients and controls were selected from medical practices in Oslo, Norway and Brussels, Belgium.

### B. Blood sampling and genotyping procedure

2 ml whole blood was collected in EDTA tubes, and stored at -80°C until analysis. Genomic DNA was extracted from 200 µl whole blood using the Blood and Tissue DNA extraction kit, Qiagen (Venlo, Netherlands). Analysis of the rs4986790, rs4986791, rs12344353, rs11209026, rs1800795 and rs2340690 polymorphisms was performed with the SNPlex™ genotyping technology, or with Taqman® SNP genotyping assays, both from Applied Biosystems (Foster City, CA, USA).

### Results

A summary of all genotyping results is provided in Table 1.

**Table 1: Frequency of specific alleles in controls and in CFS patients**

| **Gene** | **TLR4** | **IL-23R** | **Hsp60 (HSPD1)** | **IL6** | **IL6 + Hsp60** |
|---|---|---|---|---|---|
| **SNP** | rs4986790 | rs11209026 | rs2340690 | rs1800795 | Rs1800795 in combination with rs2340690 |
| | rs12344353 | | | | |
| **Allele** | G allele of rs4986790 | A allele | T/T genotype | G/G genotype | T/T genotype of rs2340690 in combination with G/G genotype of rs1800795 |
| | C allele of rs12344353 | | | | |
| **Frequency in controls** | 9% | 0% | 54% | 13% | 4% |
| **Frequency in patients** | 13% | 10% | 74% | 29% | 32% |

### A. Toll-like receptor 4

Three different SNPs have been analyzed for this gene: rs4986790 (Asp299Gly), rs4986791 (Thr399IIe) and rs12344353.

100% cosegregation level for the rs4986790 and rs12344353 SNPs was observed. rs4986791 cosegregrates with the two others, but less systematically (80% only). These high cosegregation levels were already described in the literature.

The G allele of rs4986790 is associated with a lower responsiveness to LPS stimulation. This is detrimental in that affected carriers are more susceptible to gram-negative infections (sepsis); on the other hand, these people tend to develop less inflammation, and seem to have less chance to develop cardiovascular disease or even Alzheimer's disease. TLR4 polymorphisms have been associated with intestinal diseases (Crohn's disease, inflammatory bowel disease), which may be explained by deficient host-bacteria interactions in the gut (Zacharowski et al. (2006) PNAS 103(16):6392-7; Franchimont et al. (2004) Gut 53(7):987-92).

The variant alleles were present in 6/64 (9%) of the controls, versus 14/107 (13%) of the patients (odds ratio 0.5). CFS patients therefore present a higher frequency of the TLR4 variant haplotype.

### B. Heat-shock protein 60

Hsp60 is produced in stress situations (oxidative stress, toxic insult); it can be released by the cells and work extra-cellularly as a pro-inflammatory molecule (it is recognized by TLR4 and leads to the proinflammatory activation of macrophages) (Visser et al. (1998) J. Infect. Dis. 177(2) :451-454). An intronic C/T substitution (rs2340690) has been described.

C/C or C/T genotypes of rs2340690 were observed in 26% of the patients versus in 46% of the controls (odds ratio 2.3). Therefore, carrying the T/T genotype of rs2340690 is a predisposing factor for CFS.

### C. IL-23R

A SNP located on the IL-23R gene, rs11209026, has already been associated with Crohn's disease (Roberts et al. (2007) Am. J. Gastroenterol 102(12):2754-61). The A allele of this polymorphism was found in 10% of CFS patients, but in none of the controls.

### D. IL-6, and IL-6 in combination with Hsp60

IL-6 polymorphism rs1800795 is another SNP of interest. A higher prevalence of the G/G genotype of rs1800795 was observed in patients than in controls (29% vs. 13%, OR=0.35), indicating that IL-6 polymorphisms are predictive of CFS independent of other polymorphisms, e.g. polymorphisms in other cytokines, e.g. IFNgamma. The G/G genotype of rs1800795 is thought to confer susceptibility to inflammation (Tischendorf et al. (2007) Int J. Immunogenet 34(6):413-8).

A very discriminant genotype is formed by the combination of IL-6 and Hsp60: the frequency of subjects simultaneously carrying the G/G genotype of rs1800795 (IL-6) and the T/T genotype of rs2340690 (Hsp60) was found to be 32% in the patient population, versus only 4% in the control population.

## Claims

1. A method of evaluating the susceptibility of a subject for Chronic Fatigue Syndrome (CFS),
said method comprising:
(a) genotyping said subject for at least a polymorphism in TLR4 to obtain a result; and
(b) employing said result to provide an evaluation of a subject for CFS.

2. The method according to Claim 1, wherein said TLR4 polymorphism is selected from rs4986790 and rs12344353 and combinations thereof.

3. The method according to Claim 2, wherein identification of a G allele of rs4986790 or a C allele of rs12344353 is predictive of susceptibility to CFS.

4. The method according to any one of Claims 1 to 3, wherein the method further comprises genotyping said subject for at least one polymorphism in at least one further gene selected from the group consisting of, Hsp60, IL-23R, IL-6 and IL-17F to obtain a result.

5. The method according to Claim 4, wherein the Hsp60 polymorphism is rs2340690.

6. The method according to Claim 4 or claim 5, wherein identification of two T alleles of rs2340690 is predictive of susceptibility to CFS.

7. The method according to Claim 4, wherein the IL-23R polymorphism is rs11209026.

8. The method according to Claim 7, wherein identification of an A allele of rs11209026 is predictive of susceptibility to CFS.

9. The method according to Claim 4, wherein the IL-6 polymorphism is rs1800795.

10. The method according to Claim 9, wherein identification of two G alleles of rs1800795 is predictive of susceptibility to CFS.

11. The method according to Claim 4, wherein the IL-17F polymorphism is rs763780.

12. The method according to Claim 11, wherein identification of a C allele of rs763780 is predictive of resistance to CFS.

13. Use of a kit in the method of Claims 1-12, the kit comprising at least one of:
(a) a primer that specifically hybridizes with a polymorphism in TLR4;and
(b) two or more primers specific for amplifying a region known to comprise a polymorphism in TLR4.

14. Use of the kit of Claim 13 in the method of Claims 1-11, wherein the kit further comprises:
one or more primers for detecting a polymorphism in at least one gene selected from: Hsp60, IL-23R, IL-6 and IL-17F, wherein the one or more primers for each selected gene are at least one of:
(a) a primer that specifically hybridizes with a polymorphism in the selected gene, and
(b) two or more primers specific for amplifying a region known to comprise a polymorphism in the selected gene.

## Patentansprüche

1. Verfahren zur Bewertung der Anfälligkeit von einem Subjekt für chronisches Erschöpfungssyndrom (CFS),
wobei das genannte Verfahren Folgendes umfasst:
(a) Genotypisierung des genannten Subjekts für mindestens einen Polymorphismus im TLR4, um ein Ergebnis zu erhalten; und
(b) Verwenden des genannten Ergebnisses, um eine Bewertung eines Subjekts für CFS bereitzustellen.

2. Verfahren nach Anspruch 1, worin der genannte TLR4-Polymorphismus aus rs4986790 und rs12344353 und Kombinationen davon ausgewählt ist.

3. Verfahren nach Anspruch 2, worin die Identifizierung eines G-Allels von rs4986790 oder eines C-Allels von rs12344353 eine Anfälligkeit für CFS vorhersagt.

4. Verfahren nach einem der Ansprüche 1 bis 3, worin das Verfahren weiter eine Genotypisierung des genannten Subjekts für mindestens einen Polymorphismus in mindestens einem weiteren Gen umfasst, das aus der Gruppe ausgewählt ist, die aus Hsp60, IL-23R, IL-6 und IL-17F besteht, um ein Ergebnis zu erhalten.

5. Verfahren nach Anspruch 4, worin der Hsp60-Polymorphismus rs2340690 ist.

6. Verfahren nach Anspruch 4 oder Anspruch 5, worin die Identifizierung von zwei T-Allelen von rs2340690 eine Anfälligkeit für CFS vorhersagt.

7. Verfahren nach Anspruch 4, worin der IL-23R-Polymorphismus rs11209026 ist.

8. Verfahren nach Anspruch 7, worin die Identifizierung eines A-Allels von rs11209026 eine Anfälligkeit für CFS vorhersagt.

9. Verfahren nach Anspruch 4, worin der IL-6-Polymorphismus rs1800795 ist.

10. Verfahren nach Anspruch 9, worin die Identifizierung von zwei G-Allelen von rs1800795 eine Anfälligkeit für CFS vorhersagt.

11. Verfahren nach Anspruch 4, worin der IL-17F-Polymorphismus rs763780 ist.

12. Verfahren nach Anspruch 11, worin die Identifizierung eines C-Allels von rs763780 eine Resistenz gegen CFS vorhersagt.

13. Verwendung eines Kits in dem Verfahren nach den Ansprüchen 1-12, wobei der Kit mindestens eines von Folgenden umfasst:
(a) eines Primers, der mit einem Polymorphismus im TLR4 spezifisch hybridisiert; und
(b) von zwei oder mehr Primern, die für die Amplifizierung einer Region, von der bekannt ist, dass sie einen Polymorphismus im TLR4 umfasst, spezifisch sind.

14. Verwendung des Kits nach Anspruch 13 in dem Verfahren nach den Ansprüchen 1-11, worin der Kit weiter Folgendes umfasst:
einen oder mehrere Primer für den Nachweis eines Polymorphismus in mindestens einem Gen, das aus Folgenden ausgewählt ist: Hsp60, IL-23R, IL-6 und IL-17F, worin der eine oder die mehreren Primer für jedes ausgewählte Gen mindestens einer von Folgenden sind:
(a) eines Primers, der mit einem Polymorphismus in dem ausgewählten Gen spezifisch hybridisiert, und
(b) von zwei oder mehr Primern, die für die Amplifizierung einer Region, von der bekannt ist, dass sie einen Polymorphismus in dem ausgewählten Gen umfasst, spezifisch sind.

## Revendications

1. Procédé d'évaluation de la susceptibilité d'un sujet au syndrome de fatigue chronique (CFS),
ledit procédé comprenant :
(a) effectuer le génotypage dudit sujet pour au moins un polymorphisme dans TLR4 pour obtenir un résultat ; et
(b) employer ledit résultat pour fournir une évaluation d'un sujet pour le CFS.

2. Procédé selon la revendication 1, dans lequel ledit polymorphisme de TLR4 est sélectionné parmi rs4986790 et rs12344353 et des combinaisons de ceux-ci.

3. Procédé selon la revendication 2, dans lequel l'identification d'un allèle G de rs4986790 ou d'un allèle C de rs12344353 est prédictive de la susceptibilité au CFS.

4. Procédé selon l'une quelconque des revendications 1 à 3, dans lequel le procédé comprend en outre le génotypage dudit sujet pour au moins un polymorphisme dans au moins un autre gène sélectionné parmi le groupe constitué de Hsp60, IL-23R, IL-6 et IL-17F pour obtenir un résultat.

5. Procédé selon la revendication 4, dans lequel le polymorphisme de Hsp60 est rs2340690.

6. Procédé selon la revendication 4 ou la revendication 5, dans lequel l'identification de deux allèles T de rs2340690 est prédictive de la susceptibilité au CFS.

7. Procédé selon la revendication 4, dans lequel le polymorphisme de IL-23R est rs11209026.

8. Procédé selon la revendication 7, dans lequel l'identification d'un allèle A de rs11209026 est prédictive de la susceptibilité au CFS.

9. Procédé selon la revendication 4, dans lequel le polymorphisme de IL-6 est rs1800795.

10. Procédé selon la revendication 9, dans lequel l'identification de deux allèles G de rs1800795 est prédictive de la susceptibilité au CFS.

11. Procédé selon la revendication 4, dans lequel le polymorphisme de IL-17F est rs763780.

12. Procédé selon la revendication 11, dans lequel l'identification d'un allèle C de rs763780 est prédictive de la résistance au CFS.

13. Utilisation d'un kit dans le procédé selon les revendications 1 à 12, le kit comprenant au moins une parmi :
(a) une amorce qui s'hybride spécifiquement avec un polymorphisme dans TLR4 ; et
(b) deux amorces ou plus spécifiques pour amplifier une région connue pour comprendre un polymorphisme dans TLR4.

14. Utilisation du kit selon la revendication 13 dans le procédé selon les revendications 1 à 11, dans laquelle le kit comprend en outre :
une ou plusieurs amorces pour détecter un polymorphisme dans au moins un gène sélectionné parmi : Hsp60, IL-23R, IL-6 et UL-17F, dans laquelle la ou les amorces pour chaque gène sélectionné sont au moins une parmi :
(a) une amorce qui s'hybride spécifiquement avec un polymorphisme dans le gène sélectionné, et
(b) deux amorces ou plus spécifiques pour amplifier une région connue pour comprendre un polymorphisme dans le gène sélectionné.


### SEQUENCE LISTING

<110> FREMONT, MARC
   DE MEIRLEIR, KENNY LEO
<120> Methods and Compositions for Evaluating
   Chronic Immune Diseases
<130> REDL-011WO
<150> 61/156,346
   <151> 2009-02-27
<160> 12
<170> FastSEQ for Windows Version 4.0
<210> 1
   <211> 5667
   <212> DNA
   <213> homo sapiens
<400> 1
   ctcttgctgt ttctttagcc actggtctgc aggcgttttc ttcttctaac ttcctctcct 60
   gtgacaaaag agataactat tagagaaaca aaagtccaga atgctaaggt tgccgctttc 120
   acttcctctc accctttagc ccagaactgc tttgaataca ccaattgctg tggggcggct 180
   cgaggaagag aagacaccag tgcctcagaa actgctcggt cagacggtga tagcgagcca 240
   cgcattcaca gggccactgc tgctcacaga agcagtgagg atgatgccag gatgatgtct 300
   gcctcgcgcc tggctgggac tctgatccca gccatggcct tcctctcctg cgtgagacca 360
   gaaagctggg agccctgcgt ggaggtggtt cctaatatta cttatcaatg catggagctg 420
   aatttctaca aaatccccga caacctcccc ttctcaacca agaacctgga cctgagcttt 480
   aatcccctga ggcatttagg cagctatagc ttcttcagtt tcccagaact gcaggtgctg 540
   gatttatcca ggtgtgaaat ccagacaatt gaagatgggg catatcagag cctaagccac 600
   ctctctacct taatattgac aggaaacccc atccagagtt tagccctggg agccttttct 660
   ggactatcaa gtttacagaa gctggtggct gtggagacaa atctagcatc tctagagaac 720
   ttccccattg gacatctcaa aactttgaaa gaacttaatg tggctcacaa tcttatccaa 780
   tctttcaaat tacctgagta tttttctaat ctgaccaatc tagagcactt ggacctttcc 840
   agcaacaaga ttcaaagtat ttattgcaca gacttgcggg ttctacatca aatgccccta 900
   ctcaatctct ctttagacct gtccctgaac cctatgaact ttatccaacc aggtgcattt 960
   aaagaaatta ggcttcataa gctgacttta agaaataatt ttgatagttt aaatgtaatg 1020
   aaaacttgta ttcaaggtct ggctggttta gaagtccatc gtttggttct gggagaattt 1080
   agaaatgaag gaaacttgga aaagtttgac aaatctgctc tagagggcct gtgcaatttg 1140
   accattgaag aattccgatt agcatactta gactactacc tcgatgatat tattgactta 1200
   tttaattgtt tgacaaatgt ttcttcattt tccctggtga gtgtgactat tgaaagggta 1260
   aaagactttt cttataattt cggatggcaa catttagaat tagttaactg taaatttgga 1320
   cagtttccca cattgaaact caaatctctc aaaaggctta ctttcacttc caacaaaggt 1380
   gggaatgctt tttcagaagt tgatctacca agccttgagt ttctagatct cagtagaaat 1440
   ggcttgagtt tcaaaggttg ctgttctcaa agtgattttg ggacaaccag cctaaagtat 1500
   ttagatctga gcttcaatgg tgttattacc atgagttcaa acttcttggg cttagaacaa 1560
   ctagaacatc tggatttcca gcattccaat ttgaaacaaa tgagtgagtt ttcagtattc 1620
   ctatcactca gaaacctcat ttaccttgac atttctcata ctcacaccag agttgctttc 1680
   aatggcatct tcaatggctt gtccagtctc gaagtcttga aaatggctgg caattctttc 1740
   caggaaaact tccttccaga tatcttcaca gagctgagaa acttgacctt cctggacctc 1800
   tctcagtgtc aactggagca gttgtctcca acagcattta actcactctc cagtcttcag 1860
   gtactaaata tgagccacaa caacttcttt tcattggata cgtttcctta taagtgtctg 1920
   aactccctcc aggttcttga ttacagtctc aatcacataa tgacttccaa aaaacaggaa 1980
   ctacagcatt ttccaagtag tctagctttc ttaaatctta ctcagaatga ctttgcttgt 2040
   acttgtgaac accagagttt cctgcaatgg atcaaggacc agaggcagct cttggtggaa 2100
   gttgaacgaa tggaatgtgc aacaccttca gataagcagg gcatgcctgt gctgagtttg 2160
   aatatcacct gtcagatgaa taagaccatc attggtgtgt cggtcctcag tgtgcttgta 2220
   gtatctgttg tagcagttct ggtctataag ttctattttc acctgatgct tcttgctggc 2280
   tgcataaagt atggtagagg tgaaaacatc tatgatgcct ttgttatcta ctcaagccag 2340
   gatgaggact gggtaaggaa tgagctagta aagaatttag aagaaggggt gcctccattt 2400
   cagctctgcc ttcactacag agactttatt cccggtgtgg ccattgctgc caacatcatc 2460
   catgaaggtt tccataaaag ccgaaaggtg attgttgtgg tgtcccagca cttcatccag 2520
   agccgctggt gtatctttga atatgagatt gctcagacct ggcagtttct gagcagtcgt 2580
   gctggtatca tcttcattgt cctgcagaag gtggagaaga ccctgctcag gcagcaggtg 2640
   gagctgtacc gccttctcag caggaacact tacctggagt gggaggacag tgtcctgggg 2700
   cggcacatct tctggagacg actcagaaaa gccctgctgg atggtaaatc atggaatcca 2760
   gaaggaacag tgggtacagg atgcaattgg caggaagcaa catctatctg aagaggaaaa 2820
   ataaaaacct cctgaggcat ttcttgccca gctgggtcca acacttgttc agttaataag 2880
   tattaaatgc tgccacatgt caggccttat gctaagggtg agtaattcca tggtgcacta 2940
   gatatgcagg gctgctaatc tcaaggagct tccagtgcag agggaataaa tgctagacta 3000
   aaatacagag tcttccaggt gggcatttca accaactcag tcaaggaacc catgacaaag 3060
   aaagtcattt caactcttac ctcatcaagt tgaataaaga cagagaaaac agaaagagac 3120
   attgttcttt tcctgagtct tttgaatgga aattgtatta tgttatagcc atcataaaac 3180
   cattttggta gttttgactg aactgggtgt tcactttttc ctttttgatt gaatacaatt 3240
   taaattctac ttgatgactg cagtcgtcaa ggggctcctg atgcaagatg ccccttccat 3300
   tttaagtctg tctccttaca gaggttaaag tctagtggct aattcctaag gaaacctgat 3360
   taacacatgc tcacaaccat cctggtcatt ctcgagcatg ttctattttt taactaatca 3420
   cccctgatat atttttattt ttatatatcc agttttcatt tttttacgtc ttgcctataa 3480
   gctaatatca taaataaggt tgtttaagac gtgcttcaaa tatccatatt aaccactatt 3540
   tttcaaggaa gtatggaaaa gtacactctg tcactttgtc actcgatgtc attccaaagt 3600
   tattgcctac taagtaatga ctgtcatgaa agcagcattg aaataatttg tttaaagggg 3660
   gcactctttt aaacgggaag aaaatttccg cttcctggtc ttatcatgga caatttgggc 3720
   tagaggcagg aaggaagtgg gatgacctca ggaggtcacc ttttcttgat tccagaaaca 3780
   tatgggctga taaacccggg gtgacctcat gaaatgagtt gcagcagaag tttatttttt 3840
   tcagaacaag tgatgtttga tggacctctg aatctcttta gggagacaca gatggctggg 3900
   atccctcccc tgtacccttc tcactgccag gagaactacg tgtgaaggta ttcaaggcag 3960
   ggagtataca ttgctgtttc ctgttgggca atgctccttg accacatttt gggaagagtg 4020
   gatgttatca ttgagaaaac aatgtgtctg gaattaatgg ggttcttata aagaaggttc 4080
   ccagaaaaga atgttcatcc agcctcctca gaaacagaac attcaagaaa aggacaatca 4140
   ggatgtcatc agggaaatga aaataaaaac cacaatgaga tatcacctta taccaggtag 4200
   aatggctact ataaaaaaat gaagtgtcat caaggatata gagaaattgg aacccttctt 4260
   cactgctgga gggaatggaa aatggtgtag ccgttatgaa aaacagtacg gaggtttctc 4320
   aaaaattaaa aatagaactg ctatatgatc cagcaatctc acttctgtat atatacccaa 4380
   aataattgaa atcagaattt caagaaaata tttacactcc catgttcatt gtggcactct 4440
   tcacaatcac tgtttccaaa gttatggaaa caacccaaat ttccattgaa aaataaatgg 4500
   acaaagaaaa tgtgcatata cgtacaatgg gatattattc agcctaaaaa aagggggaat 4560
   cctgttattt atgacaacat gaataaaccc ggaggccatt atgctatgta aaatgagcaa 4620
   gtaacagaaa gacaaatact gcctgatttc atttatatga ggttctaaaa tagtcaaact 4680
   catagaagca gagaatagaa cagtggttcc tagggaaaag gaggaaggga gaaatgagga 4740
   aatagggagt tgtctaattg gtataaaatt atagtatgca agatgaatta gctctaaaga 4800
   tcagctgtat agcagagttc gtataatgaa caatactgta ttatgcactt aacattttgt 4860
   taagagggta cctctcatgt taagtgttct taccatatac atatacacaa ggaagctttt 4920
   ggaggtgatg gatatattta ttaccttgat tgtggtgatg gtttgacagg tatgtgacta 4980
   tgtctaaact catcaaattg tatacattaa atatatgcag ttttataata tcaattatgt 5040
   ctgaatgaag ctataaaaaa gaaaagacaa caaaattcag ttgtcaaaac tggaaatatg 5100
   accacagtca gaagtgtttg ttactgagtg tttcagagtg tgtttggttt gagcaggtct 5160
   agggtgattg aacatccctg ggtgtgtttc catgtctcat gtactagtga aagtagatgt 5220
   gtgcatttgt gcacatatcc ctatgtatcc ctatcagggc tgtgtgtatt tgaaagtgtg 5280
   tgtgtccgca tgatcatatc tgtatagaag agagtgtgat tatatttctt gaagaataca 5340
   tccatttgaa atggatgtct atggctgttt gagatgagtt ctctactctt gtgcttgtac 5400
   agtagtctcc ccttatccct tatgcttggt ggatacgttc ttagacccca agtggatctc 5460
   tgagaccgca gatggtacca aacctcatat atgcaatatt ttttcctata cataaatacc 5520
   taagataaag ttcatcttct gaattaggca cagtaagaga ttaacaataa ctaacaataa 5580
   aattgaatag ttataataat atattgtaat aaaagttatg tgaatgtgat ctctttcttt 5640
   ctctctctca aaaaaaaaaa aaaaaaa                                    5667
<210> 2
   <211> 839
   <212> PRT
   <213> homo sapiens
<400> 2
   Met Met Ser Ala Ser Arg Leu Ala Gly Thr Leu Ile Pro Ala Met Ala
    1               5                  10                  15
   Phe Leu Ser Cys Val Arg Pro Glu Ser Trp Glu Pro Cys Val Glu Val
               20                  25                  30
   Val Pro Asn Ile Thr Tyr Gln Cys Met Glu Leu Asn Phe Tyr Lys Ile
           35                  40                  45
   Pro Asp Asn Leu Pro Phe Ser Thr Lys Asn Leu Asp Leu Ser Phe Asn
       50                  55                  60
   Pro Leu Arg His Leu Gly Ser Tyr Ser Phe Phe Ser Phe Pro Glu Leu
   65                  70                  75                  80
   Gln Val Leu Asp Leu Ser Arg Cys Glu Ile Gln Thr Ile Glu Asp Gly
                   85                  90                  95
   Ala Tyr Gln Ser Leu Ser His Leu Ser Thr Leu Ile Leu Thr Gly Asn
                           100                     105                     110
   Pro Ile Gln Ser Leu Ala Leu Gly Ala Phe Ser Gly Leu Ser Ser Leu
               115                     120                     125
   Gln Lys Leu Val Ala Val Glu Thr Asn Leu Ala Ser Leu Glu Asn Phe
           130                     135                     140
   Pro Ile Gly His Leu Lys Thr Leu Lys Glu Leu Asn Val Ala His Asn
   145                     150                     155                     160
   Leu Ile Gln Ser Phe Lys Leu Pro Glu Tyr Phe Ser Asn Leu Thr Asn
                   165                     170                     175
   Leu Glu His Leu Asp Leu Ser Ser Asn Lys Ile Gln Ser Ile Tyr Cys
               180                     185                     190
   Thr Asp Leu Arg Val Leu His Gln Met Pro Leu Leu Asn Leu Ser Leu
           195                     200                     205
   Asp Leu Ser Leu Asn Pro Met Asn Phe Ile Gln Pro Gly Ala Phe Lys
       210                     215                     220
   Glu Ile Arg Leu His Lys Leu Thr Leu Arg Asn Asn Phe Asp Ser Leu
   225                     230                     235                     240
   Asn Val Met Lys Thr Cys Ile Gln Gly Leu Ala Gly Leu Glu Val His
                   245                     250                     255
   Arg Leu Val Leu Gly Glu Phe Arg Asn Glu Gly Asn Leu Glu Lys Phe
               260                     265                     270
   Asp Lys Ser Ala Leu Glu Gly Leu Cys Asn Leu Thr Ile Glu Glu Phe
           275                     280                     285
   Arg Leu Ala Tyr Leu Asp Tyr Tyr Leu Asp Asp Ile Ile Asp Leu Phe
       290                     295                     300
   Asn Cys Leu Thr Asn Val Ser Ser Phe Ser Leu Val Ser Val Thr Ile
   305                     310                     315                     320
   Glu Arg Val Lys Asp Phe Ser Tyr Asn Phe Gly Trp Gln His Leu Glu
                   325                     330                     335
   Leu Val Asn Cys Lys Phe Gly Gln Phe Pro Thr Leu Lys Leu Lys Ser
               340                     345                     350
   Leu Lys Arg Leu Thr Phe Thr Ser Asn Lys Gly Gly Asn Ala Phe Ser
           355                     360                     365
   Glu Val Asp Leu Pro Ser Leu Glu Phe Leu Asp Leu Ser Arg Asn Gly
       370                     375                     380
   Leu Ser Phe Lys Gly Cys Cys Ser Gln Ser Asp Phe Gly Thr Thr Ser
   385                     390                     395                     400
   Leu Lys Tyr Leu Asp Leu Ser Phe Asn Gly Val Ile Thr Met Ser Ser
                   405                     410                     415
   Asn Phe Leu Gly Leu Glu Gln Leu Glu His Leu Asp Phe Gln His Ser
               420                     425                     430
   Asn Leu Lys Gln Met Ser Glu Phe Ser Val Phe Leu Ser Leu Arg Asn
           435                     440                     445
   Leu Ile Tyr Leu Asp Ile Ser His Thr His Thr Arg Val Ala Phe Asn
       450                     455                     460
   Gly Ile Phe Asn Gly Leu Ser Ser Leu Glu Val Leu Lys Met Ala Gly
   465                     470                     475                     480
   Asn Ser Phe Gln Glu Asn Phe Leu Pro Asp Ile Phe Thr Glu Leu Arg
                   485                     490                     495
   Asn Leu Thr Phe Leu Asp Leu Ser Gln Cys Gln Leu Glu Gln Leu Ser
               500                     505                     510
   Pro Thr Ala Phe Asn Ser Leu Ser Ser Leu Gln Val Leu Asn Met Ser
           515                     520                     525
   His Asn Asn Phe Phe Ser Leu Asp Thr Phe Pro Tyr Lys Cys Leu Asn
       530                     535                     540
   Ser Leu Gln Val Leu Asp Tyr Ser Leu Asn His Ile Met Thr Ser Lys
   545                     550                     555                     560
   Lys Gln Glu Leu Gln His Phe Pro Ser Ser Leu Ala Phe Leu Asn Leu
                   565                     570                     575
   Thr Gln Asn Asp Phe Ala Cys Thr Cys Glu His Gln Ser Phe Leu Gln
               580                     585                     590
   Trp Ile Lys Asp Gln Arg Gln Leu Leu Val Glu Val Glu Arg Met Glu
           595                     600                     605
   Cys Ala Thr Pro Ser Asp Lys Gln Gly Met Pro Val Leu Ser Leu Asn
       610                     615                     620
   Ile Thr Cys Gln Met Asn Lys Thr Ile Ile Gly Val Ser Val Leu Ser
   625                     630                     635                     640
   Val Leu Val Val Ser Val Val Ala Val Leu Val Tyr Lys Phe Tyr Phe
                   645                     650                     655
   His Leu Met Leu Leu Ala Gly Cys Ile Lys Tyr Gly Arg Gly Glu Asn
               660                     665                     670
   Ile Tyr Asp Ala Phe Val Ile Tyr Ser Ser Gln Asp Glu Asp Trp Val
   675 680 685
   Arg Asn Glu Leu Val Lys Asn Leu Glu Glu Gly Val Pro Pro Phe Gln
   690 695 700
   Leu Cys Leu His Tyr Arg Asp Phe Ile Pro Gly Val Ala Ile Ala Ala
   705 710 715 720
   Asn Ile Ile His Glu Gly Phe His Lys Ser Arg Lys Val Ile Val Val
   725 730 735
   Val Ser Gln His Phe Ile Gln Ser Arg Trp Cys Ile Phe Glu Tyr Glu
   740 745 750
   Ile Ala Gln Thr Trp Gln Phe Leu Ser Ser Arg Ala Gly Ile Ile Phe
   755 760 765
   Ile Val Leu Gln Lys Val Glu Lys Thr Leu Leu Arg Gln Gln Val Glu
   770 775 780
   Leu Tyr Arg Leu Leu Ser Arg Asn Thr Tyr Leu Glu Trp Glu Asp Ser
   785 790 795 800
   Val Leu Gly Arg His Ile Phe Trp Arg Arg Leu Arg Lys Ala Leu Leu
   805 810 815
   Asp Gly Lys Ser Trp Asn Pro Glu Gly Thr Val Gly Thr Gly Cys Asn
   820 825 830
   Trp Gln Glu Ala Thr Ser Ile
   835
<210> 3
   <211> 2339
   <212> DNA
   <213> homo sapiens
<400> 3
   acgcggtgcc gcggggcggg agtagaggcg gagggagggg acacgggctc attgcggtgt 60
   gcgccctgca ctctgtccct cactcgccgc cgacgacctg tctcgccgag cgcacgcctt 120
   gccgccgccc cgcagaaatg cttcggttac ccacagtctt tcgccagatg agaccggtgt 180
   ccagggtact ggctcctcat ctcactcggg cttatgccaa agatgtaaaa tttggtgcag 240
   atgcccgagc cttaatgctt caaggtgtag accttttagc cgatgctgtg gccgttacaa 300
   tggggccaaa gggaagaaca gtgattattg agcagagttg gggaagtccc aaagtaacaa 360
   aagatggtgt gactgttgca aagtcaattg acttaaaaga taaatacaaa aacattggag 420
   ctaaacttgt tcaagatgtt gccaataaca caaatgaaga agctggggat ggcactacca 480
   ctgctactgt actggcacgc tctatagcca aggaaggctt cgagaagatt agcaaaggtg 540
   ctaatccagt ggaaatcagg agaggtgtga tgttagctgt tgatgctgta attgctgaac 600
   ttaaaaagca gtctaaacct gtgaccaccc ctgaagaaat tgcacaggtt gctacgattt 660
   ctgcaaacgg agacaaagaa attggcaata tcatctctga tgcaatgaaa aaagttggaa 720
   gaaagggtgt catcacagta aaggatggaa aaacactgaa tgatgaatta gaaattattg 780
   aaggcatgaa gtttgatcga ggctatattt ctccatactt tattaataca tcaaaaggtc 840
   agaaatgtga attccaggat gcctatgttc tgttgagtga aaagaaaatt tctagtatcc 900
   agtccattgt acctgctctt gaaattgcca atgctcaccg taagcctttg gtcataatcg 960
   ctgaagatgt tgatggagaa gctctaagta cactcgtctt gaataggcta aaggttggtc 1020
   ttcaggttgt ggcagtcaag gctccagggt ttggtgacaa tagaaagaac cagcttaaag 1080
   atatggctat tgctactggt ggtgcagtgt ttggagaaga gggattgacc ctgaatcttg 1140
   aagacgttca gcctcatgac ttaggaaaag ttggagaggt cattgtgacc aaagacgatg 1200
   ccatgctctt aaaaggaaaa ggtgacaagg ctcaaattga aaaacgtatt caagaaatca 1260
   ttgagcagtt agatgtcaca actagtgaat atgaaaagga aaaactgaat gaacggcttg 1320
   caaaactttc agatggagtg gctgtgctga aggttggtgg gacaagtgat gttgaagtga 1380
   atgaaaagaa agacagagtt acagatgccc ttaatgctac aagagctgct gttgaagaag 1440
   gcattgtttt gggagggggt tgtgccctcc ttcgatgcat tccagccttg gactcattga 1500
   ctccagctaa tgaagatcaa aaaattggta tagaaattat taaaagaaca ctcaaaattc 1560
   cagcaatgac cattgctaag aatgcaggtg ttgaaggatc tttgatagtt gagaaaatta 1620
   tgcaaagttc ctcagaagtt ggttatgatg ctatggctgg agattttgtg aatatggtgg 1680
   aaaaaggaat cattgaccca acaaaggttg tgagaactgc tttattggat gctgctggtg 1740
   tggcctctct gttaactaca gcagaagttg tagtcacaga aattcctaaa gaagagaagg 1800
   accctggaat gggtgcaatg ggtggaatgg gaggtggtat gggaggtggc atgttctaac 1860
   tcctagacta gtgctttacc tttattaatg aactgtgaca ggaagcccaa ggcagtgttc 1920
   ctcaccaata acttcagaga agtcagttgg agaaaatgaa gaaaaaggct ggctgaaaat 1980
   cactataacc atcagttact ggtttcagtt gacaaaatat ataatggttt actgctgtca 2040
   ttgtccatgc ctacagataa tttattttgt atttttgaat aaaaaacatt tgtacattcc 2100
   tgatactggg tacaagagcc atgtaccagt gtactgcttt caacttaaat cactgaggca 2160
   tttttactac tattctgtta aaatcaggat tttagtgctt gccaccacca gatgagaagt 2220
   taagcagcct ttctgtggag agtgagaata attgtgtaca aagtagagaa gtatccaatt 2280
   atgtgacaac ctttgtgtaa taaaaatttg tttaaagtta aaaaaaaaaa aaaaaaaaa  2339
<210> 4
   <211> 573
   <212> PRT
   <213> homo sapiens
<400> 4
<210> 5
   <211> 2319
   <212> DNA
   <213> homo sapiens
<400> 5
   gccccgacgc gcaccgcgat tcgccccaag ggccctgcgc aggacgctga cgcgaagact 60
   cggaggcgga agaaaaaagg agctgtttct aggcttttct aggcgcccag ccgagaaatg 120
   cttcggttac ccacagtctt tcgccagatg agaccggtgt ccagggtact ggctcctcat 180
   ctcactcggg cttatgccaa agatgtaaaa tttggtgcag atgcccgagc cttaatgctt 240
   caaggtgtag accttttagc cgatgctgtg gccgttacaa tggggccaaa gggaagaaca 300
   gtgattattg agcagagttg gggaagtccc aaagtaacaa aagatggtgt gactgttgca 360
   aagtcaattg acttaaaaga taaatacaaa aacattggag ctaaacttgt tcaagatgtt 420
   gccaataaca caaatgaaga agctggggat ggcactacca ctgctactgt actggcacgc 480
   tctatagcca aggaaggctt cgagaagatt agcaaaggtg ctaatccagt ggaaatcagg 540
   agaggtgtga tgttagctgt tgatgctgta attgctgaac ttaaaaagca gtctaaacct 600
   gtgaccaccc ctgaagaaat tgcacaggtt gctacgattt ctgcaaacgg agacaaagaa 660
   attggcaata tcatctctga tgcaatgaaa aaagttggaa gaaagggtgt catcacagta 720
   aaggatggaa aaacactgaa tgatgaatta gaaattattg aaggcatgaa gtttgatcga 780
   ggctatattt ctccatactt tattaataca tcaaaaggtc agaaatgtga attccaggat 840
   gcctatgttc tgttgagtga aaagaaaatt tctagtatcc agtccattgt acctgctctt 900
   gaaattgcca atgctcaccg taagcctttg gtcataatcg ctgaagatgt tgatggagaa 960
   gctctaagta cactcgtctt gaataggcta aaggttggtc ttcaggttgt ggcagtcaag 1020
   gctccagggt ttggtgacaa tagaaagaac cagcttaaag atatggctat tgctactggt 1080
   ggtgcagtgt ttggagaaga gggattgacc ctgaatcttg aagacgttca gcctcatgac 1140
   ttaggaaaag ttggagaggt cattgtgacc aaagacgatg ccatgctctt aaaaggaaaa 1200
   ggtgacaagg ctcaaattga aaaacgtatt caagaaatca ttgagcagtt agatgtcaca 1260
   actagtgaat atgaaaagga aaaactgaat gaacggcttg caaaactttc agatggagtg 1320
   gctgtgctga aggttggtgg gacaagtgat gttgaagtga atgaaaagaa agacagagtt 1380
   acagatgccc ttaatgctac aagagctgct gttgaagaag gcattgtttt gggagggggt 1440
   tgtgccctcc ttcgatgcat tccagccttg gactcattga ctccagctaa tgaagatcaa 1500
   aaaattggta tagaaattat taaaagaaca ctcaaaattc cagcaatgac cattgctaag 1560
   aatgcaggtg ttgaaggatc tttgatagtt gagaaaatta tgcaaagttc ctcagaagtt 1620
   ggttatgatg ctatggctgg agattttgtg aatatggtgg aaaaaggaat cattgaccca 1680
   acaaaggttg tgagaactgc tttattggat gctgctggtg tggcctctct gttaactaca 1740
   gcagaagttg tagtcacaga aattcctaaa gaagagaagg accctggaat gggtgcaatg 1800
   ggtggaatgg gaggtggtat gggaggtggc atgttctaac tcctagacta gtgctttacc 1860
   tttattaatg aactgtgaca ggaagcccaa ggcagtgttc ctcaccaata acttcagaga 1920
   agtcagttgg agaaaatgaa gaaaaaggct ggctgaaaat cactataacc atcagttact 1980
   ggtttcagtt gacaaaatat ataatggttt actgctgtca ttgtccatgc ctacagataa 2040
   tttattttgt atttttgaat aaaaaacatt tgtacattcc tgatactggg tacaagagcc 2100
   atgtaccagt gtactgcttt caacttaaat cactgaggca tttttactac tattctgtta 2160
   aaatcaggat tttagtgctt gccaccacca gatgagaagt taagcagcct ttctgtggag 2220
   agtgagaata attgtgtaca aagtagagaa gtatccaatt atgtgacaac ctttgtgtaa 2280
   taaaaatttg tttaaagtta aaaaaaaaaa aaaaaaaaa                        2319
<210> 6
   <211> 573
   <212> PRT
   <213> homo sapiens
<400> 6
<210> 7
   <211> 2826
   <212> DNA
   <213> homo sapiens
<400> 7
   acaagggtgg cagcctggct ctgaagtgga attatgtgct tcaaacaggt tgaaagaggg 60
   aaacagtctt ttcctgcttc cagacatgaa tcaggtcact attcaatggg atgcagtaat 120
   agccctttac atactcttca gctggtgtca tggaggaatt acaaatataa actgctctgg 180
   ccacatctgg gtagaaccag ccacaatttt taagatgggt atgaatatct ctatatattg 240
   ccaagcagca attaagaact gccaaccaag gaaacttcat ttttataaaa atggcatcaa 300
   agaaagattt caaatcacaa ggattaataa aacaacagct cggctttggt ataaaaactt 360
   tctggaacca catgcttcta tgtactgcac tgctgaatgt cccaaacatt ttcaagagac 420
   actgatatgt ggaaaagaca tttcttctgg atatccgcca gatattcctg atgaagtaac 480
   ctgtgtcatt tatgaatatt caggcaacat gacttgcacc tggaatgctg ggaagctcac 540
   ctacatagac acaaaatacg tggtacatgt gaagagttta gagacagaag aagagcaaca 600
   gtatctcacc tcaagctata ttaacatctc cactgattca ttacaaggtg gcaagaagta 660
   cttggtttgg gtccaagcag caaacgcact aggcatggaa gagtcaaaac aactgcaaat 720
   tcacctggat gatatagtga taccttctgc agccgtcatt tccagggctg agactataaa 780
   tgctacagtg cccaagacca taatttattg ggatagtcaa acaacaattg aaaaggtttc 840
   ctgtgaaatg agatacaagg ctacaacaaa ccaaacttgg aatgttaaag aatttgacac 900
   caattttaca tatgtgcaac agtcagaatt ctacttggag ccaaacatta agtacgtatt 960
   tcaagtgaga tgtcaagaaa caggcaaaag gtactggcag ccttggagtt cactgttttt 1020
   tcataaaaca cctgaaacag ttccccaggt cacatcaaaa gcattccaac atgacacatg 1080
   gaattctggg ctaacagttg cttccatctc tacagggcac cttacttctg acaacagagg 1140
   agacattgga cttttattgg gaatgatcgt ctttgctgtt atgttgtcaa ttctttcttt 1200
   gattgggata tttaacagat cattccgaac tgggattaaa agaaggatct tattgttaat 1260
   accaaagtgg ctttatgaag atattcctaa tatgaaaaac agcaatgttg tgaaaatgct 1320
   acaggaaaat agtgaactta tgaataataa ttccagtgag caggtcctat atgttgatcc 1380
   catgattaca gagataaaag aaatcttcat cccagaacac aagcctacag actacaagaa 1440
   ggagaataca ggacccctgg agacaagaga ctacccgcaa aactcgctat tcgacaatac 1500
   tacagttgta tatattcctg atctcaacac tggatataaa ccccaaattt caaattttct 1560
   gcctgaggga agccatctca gcaataataa tgaaattact tccttaacac ttaaaccacc 1620
   agttgattcc ttagactcag gaaataatcc caggttacaa aagcatccta attttgcttt 1680
   ttctgtttca agtgtgaatt cactaagcaa cacaatattt cttggagaat taagcctcat 1740
   attaaatcaa ggagaatgca gttctcctga catacaaaac tcagtagagg aggaaaccac 1800
   catgcttttg gaaaatgatt cacccagtga aactattcca gaacagaccc tgcttcctga 1860
   tgaatttgtc tcctgtttgg ggatcgtgaa tgaggagttg ccatctatta atacttattt 1920
   tccacaaaat attttggaaa gccacttcaa taggatttca ctcttggaaa agtagagctg 1980
   tgtggtcaaa atcaatatga gaaagctgcc ttgcaatctg aacttgggtt ttccctgcaa 2040
   tagaaattga attctgcctc tttttgaaaa aaatgtattc acatacaaat cttcacatgg 2100
   acacatgttt tcatttccct tggataaata cctaggtagg ggattgctgg gccatatgat 2160
   aagcatatgt ttcagttcta ccaatcttgt ttccagagta gtgacatttc tgtgctccta 2220
   ccatcaccat gtaagaattc ccgggagctc catgcctttt taattttagc cattcttctg 2280
   cctcatttct taaaattaga gaattaaggt cccgaaggtg gaacatgctt catggtcaca 2340
   catacaggca caaaaacagc attatgtgga cgcctcatgt attttttata gagtcaacta 2400
   tttcctcttt attttccctc attgaaagat gcaaaacagc tctctattgt gtacagaaag 2460
   ggtaaataat gcaaaatacc tggtagtaaa ataaatgctg aaaattttcc tttaaaatag 2520
   aatcattagg ccaggcgtgg tggctcatgc ttgtaatccc agcactttgg taggctgagg 2580
   taggtggatc acctgaggtc aggagttcga gtccagcctg gccaatatgc tgaaaccctg 2640
   tctctactaa aattacaaaa attagccggc catggtggca ggtgcttgta atcccagcta 2700
   cttgggaggc tgaggcagga gaatcacttg aaccaggaag gcagaggttg cactgagctg 2760
   agattgtgcc actgcactcc agcctgggca acaagagcaa aactctgtct ggaaaaaaaa 2820
   aaaaaa 2826
<210> 8
   <211> 629
   <212> PRT
   <213> homo sapiens
<400> 8
   Met Asn Gln Val Thr Ile Gln Trp Asp Ala Val Ile Ala Leu Tyr Ile
    1               5                  10                  15
   Leu Phe Ser Trp Cys His Gly Gly Ile Thr Asn Ile Asn Cys Ser Gly
               20                  25                  30
   His Ile Trp Val Glu Pro Ala Thr Ile Phe Lys Met Gly Met Asn Ile
           35                  40                  45
   Ser Ile Tyr Cys Gln Ala Ala Ile Lys Asn Cys Gln Pro Arg Lys Leu
       50                  55                  60
   His Phe Tyr Lys Asn Gly Ile Lys Glu Arg Phe Gln Ile Thr Arg Ile
   65                  70                  75                  80
   Asn Lys Thr Thr Ala Arg Leu Trp Tyr Lys Asn Phe Leu Glu Pro His
                   85                  90                  95
   Ala Ser Met Tyr Cys Thr Ala Glu Cys Pro Lys His Phe Gln Glu Thr
               100                 105                 110
   Leu Ile Cys Gly Lys Asp Ile Ser Ser Gly Tyr Pro Pro Asp Ile Pro
           115                 120                 125
   Asp Glu Val Thr Cys Val Ile Tyr Glu Tyr Ser Gly Asn Met Thr Cys
       130                 135                 140
   Thr Trp Asn Ala Gly Lys Leu Thr Tyr Ile Asp Thr Lys Tyr Val Val
   145                 150                 155                 160
   His Val Lys Ser Leu Glu Thr Glu Glu Glu Gln Gln Tyr Leu Thr Ser
                   165                 170                 175
   Ser Tyr Ile Asn Ile Ser Thr Asp Ser Leu Gln Gly Gly Lys Lys Tyr
               180                 185                 190
   Leu Val Trp Val Gln Ala Ala Asn Ala Leu Gly Met Glu Glu Ser Lys
           195                 200                 205
   Gln Leu Gln Ile His Leu Asp Asp Ile Val Ile Pro Ser Ala Ala Val
       210                 215                 220
   Ile Ser Arg Ala Glu Thr Ile Asn Ala Thr Val Pro Lys Thr Ile Ile
   225                 230                 235                 240
   Tyr Trp Asp Ser Gln Thr Thr Ile Glu Lys Val Ser Cys Glu Met Arg
                   245                 250                 255
   Tyr Lys Ala Thr Thr Asn Gln Thr Trp Asn Val Lys Glu Phe Asp Thr
               260                 265                 270
   Asn Phe Thr Tyr Val Gln Gln Ser Glu Phe Tyr Leu Glu Pro Asn Ile
           275                 280                 285
   Lys Tyr Val Phe Gln Val Arg Cys Gln Glu Thr Gly Lys Arg Tyr Trp
       290                 295                 300
   Gln Pro Trp Ser Ser Leu Phe Phe His Lys Thr Pro Glu Thr Val Pro
   305                 310                 315                 320
   Gln Val Thr Ser Lys Ala Phe Gln His Asp Thr Trp Asn Ser Gly Leu
                   325                 330                 335
   Thr Val Ala Ser Ile Ser Thr Gly His Leu Thr Ser Asp Asn Arg Gly
               340                 345                 350
   Asp Ile Gly Leu Leu Leu Gly Met Ile Val Phe Ala Val Met Leu Ser
           355                 360                 365
   Ile Leu Ser Leu Ile Gly Ile Phe Asn Arg Ser Phe Arg Thr Gly Ile
       370                 375                 380
   Lys Arg Arg Ile Leu Leu Leu Ile Pro Lys Trp Leu Tyr Glu Asp Ile
   385                 390                 395                 400
   Pro Asn Met Lys Asn Ser Asn Val Val Lys Met Leu Gln Glu Asn Ser
                   405                 410                 415
   Glu Leu Met Asn Asn Asn Ser Ser Glu Gln Val Leu Tyr Val Asp Pro
               420                 425                 430
   Met Ile Thr Glu Ile Lys Glu Ile Phe Ile Pro Glu His Lys Pro Thr
           435                 440                 445
   Asp Tyr Lys Lys Glu Asn Thr Gly Pro Leu Glu Thr Arg Asp Tyr Pro
       450                 455                 460
   Gln Asn Ser Leu Phe Asp Asn Thr Thr Val Val Tyr Ile Pro Asp Leu
   465                 470                 475                 480
   Asn Thr Gly Tyr Lys Pro Gln Ile Ser Asn Phe Leu Pro Glu Gly Ser
                   485                 490                 495
   His Leu Ser Asn Asn Asn Glu Ile Thr Ser Leu Thr Leu Lys Pro Pro
               500                 505                 510
   Val Asp Ser Leu Asp Ser Gly Asn Asn Pro Arg Leu Gln Lys His Pro
           515                 520                 525
   Asn Phe Ala Phe Ser Val Ser Ser Val Asn Ser Leu Ser Asn Thr Ile
       530                 535                 540
   Phe Leu Gly Glu Leu Ser Leu Ile Leu Asn Gln Gly Glu Cys Ser Ser
   545                 550                 555                 560
   Pro Asp Ile Gln Asn Ser Val Glu Glu Glu Thr Thr Met Leu Leu Glu
                   565                 570                 575
   Asn Asp Ser Pro Ser Glu Thr Ile Pro Glu Gln Thr Leu Leu Pro Asp
               580                 585                 590
   Glu Phe Val Ser Cys Leu Gly Ile Val Asn Glu Glu Leu Pro Ser Ile
           595                 600                 605
   Asn Thr Tyr Phe Pro Gln Asn Ile Leu Glu Ser His Phe Asn Arg Ile
       610                 615                 620
   Ser Leu Leu Glu Lys
   625
<210> 9
   <211> 1201
   <212> DNA
   <213> homo sapiens
<400> 9
   aatattagag tctcaacccc caataaatat aggactggag atgtctgagg ctcattctgc 60
   cctcgagccc accgggaacg aaagagaagc tctatctccc ctccaggagc ccagctatga 120
   actccttctc cacaagcgcc ttcggtccag ttgccttctc cctggggctg ctcctggtgt 180
   tgcctgctgc cttccctgcc ccagtacccc caggagaaga ttccaaagat gtagccgccc 240
   cacacagaca gccactcacc tcttcagaac gaattgacaa acaaattcgg tacatcctcg 300
   acggcatctc agccctgaga aaggagacat gtaacaagag taacatgtgt gaaagcagca 360
   aagaggcact ggcagaaaac aacctgaacc ttccaaagat ggctgaaaaa gatggatgct 420
   tccaatctgg attcaatgag gagacttgcc tggtgaaaat catcactggt cttttggagt 480
   ttgaggtata cctagagtac ctccagaaca gatttgagag tagtgaggaa caagccagag 540
   ctgtgcagat gagtacaaaa gtcctgatcc agttcctgca gaaaaaggca aagaatctag 600
   atgcaataac cacccctgac ccaaccacaa atgccagcct gctgacgaag ctgcaggcac 660
   agaaccagtg gctgcaggac atgacaactc atctcattct gcgcagcttt aaggagttcc 720
   tgcagtccag cctgagggct cttcggcaaa tgtagcatgg gcacctcaga ttgttgttgt 780
   taatgggcat tccttcttct ggtcagaaac ctgtccactg ggcacagaac ttatgttgtt 840
   ctctatggag aactaaaagt atgagcgtta ggacactatt ttaattattt ttaatttatt 900
   aatatttaaa tatgtgaagc tgagttaatt tatgtaagtc atatttatat ttttaagaag 960
   taccacttga aacattttat gtattagttt tgaaataata atggaaagtg gctatgcagt 1020
   ttgaatatcc tttgtttcag agccagatca tttcttggaa agtgtaggct tacctcaaat 1080
   aaatggctaa cttatacata tttttaaaga aatatttata ttgtatttat ataatgtata 1140
   aatggttttt ataccaataa atggcatttt aaaaaattca gcaaaaaaaa aaaaaaaaaa 1200
   a                                                                 1201
<210> 10
   <211> 212
   <212> PRT
   <213> homo sapiens
<400> 10
   Met Asn Ser Phe Ser Thr Ser Ala Phe Gly Pro Val Ala Phe Ser Leu
    1               5                  10                  15
   Gly Leu Leu Leu Val Leu Pro Ala Ala Phe Pro Ala Pro Val Pro Pro
               20                  25                  30
   Gly Glu Asp Ser Lys Asp Val Ala Ala Pro His Arg Gln Pro Leu Thr
           35                  40                  45
   Ser Ser Glu Arg Ile Asp Lys Gln Ile Arg Tyr Ile Leu Asp Gly Ile
       50                  55                  60
   Ser Ala Leu Arg Lys Glu Thr Cys Asn Lys Ser Asn Met Cys Glu Ser
   65                  70                  75                  80
   Ser Lys Glu Ala Leu Ala Glu Asn Asn Leu Asn Leu Pro Lys Met Ala
                   85                  90                  95
   Glu Lys Asp Gly Cys Phe Gln Ser Gly Phe Asn Glu Glu Thr Cys Leu
               100                 105                 110
   Val Lys Ile Ile Thr Gly Leu Leu Glu Phe Glu Val Tyr Leu Glu Tyr
           115                 120                 125
   Leu Gln Asn Arg Phe Glu Ser Ser Glu Glu Gln Ala Arg Ala Val Gln
       130                 135                 140
   Met Ser Thr Lys Val Leu Ile Gln Phe Leu Gln Lys Lys Ala Lys Asn
   145                 150                 155                 160
   Leu Asp Ala Ile Thr Thr Pro Asp Pro Thr Thr Asn Ala Ser Leu Leu
                   165                 170                 175
   Thr Lys Leu Gln Ala Gln Asn Gln Trp Leu Gln Asp Met Thr Thr His
               180                 185                 190
   Leu Ile Leu Arg Ser Phe Lys Glu Phe Leu Gln Ser Ser Leu Arg Ala
           195                 200                 205
   Leu Arg Gln Met
       210
<210> 11
   <211> 808
   <212> DNA
   <213> homo sapiens
<400> 11
   gaacacaggc atacacagga agatacattc acagaaagag cttcctgcac aaagtaagcc 60
   accagcgcaa catgacagtg aagaccctgc atggcccagc catggtcaag tacttgctgc 120
   tgtcgatatt ggggcttgcc tttctgagtg aggcggcagc tcggaaaatc cccaaagtag 180
   gacatacttt tttccaaaag cctgagagtt gcccgcctgt gccaggaggt agtatgaagc 240
   ttgacattgg catcatcaat gaaaaccagc gcgtttccat gtcacgtaac atcgagagcc 300
   gctccacctc cccctggaat tacactgtca cttgggaccc caaccggtac ccctcggaag 360
   ttgtacaggc ccagtgtagg aacttgggct gcatcaatgc tcaaggaaag gaagacatct 420
   ccatgaattc cgttcccatc cagcaagaga ccctggtcgt ccggaggaag caccaaggct 480
   gctctgtttc tttccagttg gagaaggtgc tggtgactgt tggctgcacc tgcgtcaccc 540
   ctgtcatcca ccatgtgcag taagaggtgc atatccactc agctgaagaa gctgtagaaa 600
   tgccactcct tacccagtgc tctgcaacaa gtcctgtctg acccccaatt ccctccactt 660
   cacaggactc ttaataagac ctgcacggat ggaaacagaa aatattcaca atgtatgtgt 720
   gtatgtacta cactttatat ttgatatcta aaatgttagg agaaaaatta atatattcag 780
   tgctaatata ataaagtatt aataattt                                    808
<210> 12
   <211> 163
   <212> PRT
   <213> homo sapiens
<400> 12
   Met Thr Val Lys Thr Leu His Gly Pro Ala Met Val Lys Tyr Leu Leu
    1              5                  10                  15
   Leu Ser Ile Leu Gly Leu Ala Phe Leu Ser Glu Ala Ala Ala Arg Lys
               20                  25                  30
   Ile Pro Lys Val Gly His Thr Phe Phe Gln Lys Pro Glu Ser Cys Pro
           35                  40                  45
   Pro Val Pro Gly Gly Ser Met Lys Leu Asp Ile Gly Ile Ile Asn Glu
       50                  55                  60
   Asn Gln Arg Val Ser Met Ser Arg Asn Ile Glu Ser Arg Ser Thr Ser
   65                  70                  75                  80
   Pro Trp Asn Tyr Thr Val Thr Trp Asp Pro Asn Arg Tyr Pro Ser Glu
                   85                  90                  95
   Val Val Gln Ala Gln Cys Arg Asn Leu Gly Cys Ile Asn Ala Gln Gly
               100                 105                 110
   Lys Glu Asp Ile Ser Met Asn Ser Val Pro Ile Gln Gln Glu Thr Leu
           115                 120                 125
   Val Val Arg Arg Lys His Gln Gly Cys Ser Val Ser Phe Gln Leu Glu
       130                 135                 140
   Lys Val Leu Val Thr Val Gly Cys Thr Cys Val Thr Pro Val Ile His
   145                 150                 155                 160
   His Val Gln